(19)

Europäisches
Patentamt
European
Patent Office
Office européen
des brevets

(11) **EP 2 592 140 A1**

(12) **EUROPEAN PATENT APPLICATION**
published in accordance with Art. 153(4) EPC

(43) Date of publication:
**15.05.2013  Bulletin 2013/20**

(21) Application number: **11792493.6**

(22) Date of filing: **08.06.2011**

(51) Int Cl.:
**C12N 11/14** *(2006.01)*        **C01B 37/00** *(2006.01)*
**C07K 17/14** *(2006.01)*

(86) International application number:
**PCT/JP2011/063160**

(87) International publication number:
**WO 2011/155536 (15.12.2011 Gazette 2011/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **09.06.2010   JP 2010132375**

(71) Applicants:
• **JGC Corporation
  Tokyo 100-0004 (JP)**
• **National Institute of Advanced Industrial Science
  and Technology
  Tokyo 100-8921 (JP)**

(72) Inventors:
• **TSUNODA, Tatsuo
  Tsukuba-shi
  Ibaraki 305-8565 (JP)**
• **NARA, Takayuki
  Tsukuba-shi
  Ibaraki 305-8565 (JP)**
• **ONO, Seigo
  Tsukuba-shi
  Ibaraki 305-8565 (JP)**

• **SEKIKAWA, Chisato
  Tsukuba-shi
  Ibaraki 305-8565 (JP)**
• **MIZUKAMI, Fujio
  Tsukuba-shi
  Ibaraki 305-8565 (JP)**
• **KOJIMA, Shuzo
  Higashiibaraki-gun
  Ibaraki 311-1313 (JP)**
• **TAHARA, Naoki
  Yokohama-shi
  Kanagawa 220-6001 (JP)**
• **TOGASHI, Hideaki
  Yokohama-shi
  Kanagawa 220-6001 (JP)**
• **EGAMI, Miki
  Kitakyushu-shi
  Fukuoka 808-0027 (JP)**

(74) Representative: **Becker Kurig Straus
Patentanwälte
Bavariastrasse 7
80336 München (DE)**

(54) **SUPPORT FOR PROTEIN IMMOBILIZATION, IMMOBILIZED PROTEIN AND METHOD FOR PRODUCING SAME**

(57)    A support for enzyme immobilization is described, which is for immobilizing enzymes of various molecular sizes and also for, due to the modification of the surface silanol groups of porous silica particles, for immobilizing various kinds of enzymes, and enables the design of an immobilized enzyme, which exhibits an activity equivalent to that of the corresponding non-immobilized enzyme and withstands repeated use. A method for producing the support is also described. The support includes porous silica particles having an inter-particle void structure therein, characterized in that the porous silica particles have a specific average particle size, a specific specific surface area, a specific pore volume, a specific pore size distribution and a specific porosity and have a substituent containing an organic group or an amino group on the surface thereof. An immobilized protein obtained by immobilizing a protein on the above support is also described.

EP 2 592 140 A1

**Description**

**FIELD**

The present invention relates to a support for protein immobilization, the support including a porous silica particle having mesopores and a particular organic group on the surface thereof, a method for producing the support for protein immobilization, an immobilized protein obtained by immobilizing a protein on the support for protein immobilization, and a method for producing the immobilized protein.

**BACKGROUND**

Reactions catalyzed by enzymes exhibit substrate specificity and stereospecificy that are not exhibited by inorganic catalysts, and thus it is expected that such reactions catalyzed by enzymes contribute to simplification of synthesis reaction routes. However, the cost of enzymes is high, and enzymes are not easily repeatedly used. Furthermore, there is a known problem that, for example, the environment in which the necessary activity can be maintained (i.e., the temperature range or the type of target substrate) is narrow. It has been already reported that this problem can be solved by immobilizing an enzyme on a support substance.

However, since enzymes have high substrate specificities, it is necessary to select an enzyme suitable for each reaction. In addition, since the tendency of immobilization of an enzyme is affected by the structure of the support, it is necessary to develop a method for immobilizing an enzyme on a support or a novel support for each enzyme.

To solve these problems, for example, an inclusion-type solidification method using a porous silica particle (folded-sheet mesoporous material (FSM)) obtained by changing a layer structure of layered silica using a surfactant or the like has been reported at the laboratory level. However, the mechanical strength of the resulting porous silica particles is low, and thus it is difficult to produce these porous silica particles on an industrial scale. Thus, a support that can inclusively immobilize various types of enzymes as in the case of the FSM and that can be produced on an industrial scale has been desired.

In the technical fields of decomposition of biomass and production of fiber or food using an enzyme etc., in general, an operation has hitherto often been performed using an enzyme in the form of an aqueous solution. However, in this operation method, since the solubility of the enzyme, which is a protein, is limited, in the case where a certain concentration or more of the enzyme is present, the enzyme may aggregate and often lose its activity. On the other hand, it is expected that, in a reaction system using an enzyme reaction, with an increase in the amount of enzyme present, the rate of reaction, and furthermore, the rate of production of a product can be increased.

Meanwhile, porous silica particles are characterized by having pores with a diameter of 2 to 50 nm, and an enzyme can be introduced or immobilized in the pores. Accordingly, porous silica particles are considered to be a promising support material having a large number of surfaces effective for adsorbing or immobilizing an enzyme, i.e., a protein, which has a size of several nanometers to several tens of nanometers.

Regarding an example of porous silica particles, for example, PTL 1 discloses an invention related to a spherical mesoporous body composed of a silica-based porous body that includes a sphere having a diameter of 2 mm or less and that has a large number of pores, wherein a central pore diameter D of the pores is in the range of 1 to 10 nm, and a total volume of pores having a pore diameter in the range of D - 2.5 nm to D + 2.5 nm is 60% or more of a total volume of all the pores.

PTL 2 discloses an invention related to a mesoporous inorganic body composed of an aggregate of flaky particles containing silica as a main component, wherein the mesoporous inorganic body has a specific surface area of 1,200 $m^2$/g or more. As for preferable conditions for the mesoporous inorganic body, it is described that the specific surface area is 1,400 $m^2$/g or more, the aspect ratio of the flaky particles is in the range of 5 to 100, and a peak half-width of the pore size distribution of the mesopores is within ±30% of a peak pore size (do).

PTL 3 discloses an invention related to porous silica aggregated particles obtained by aggregating porous silica spherical primary particles that exhibit an X-ray diffraction pattern having at least one diffraction line in a range of a diffraction angle (2θ/°) corresponding to a lattice spacing (d) of 1 nm or more, wherein pores are formed in the porous silica spherical primary particles and a gap layer is formed in the gaps between the porous silica spherical primary particles. This invention is common to the invention disclosed in PTL 2 in that porous silica particles are formed by aggregation of silica fine particles (primary particles).

As an example of a mesoporous body including a porous particle composed of aggregates of particles, in addition to the above examples, PTL 4 discloses an oxide porous body, though this porous body is composed of alumina. Specifically, PTL 4 discloses an alumina-based porous body in which a central pore size is within a range of 2 to 100 nm of a mesopore region. Regarding the distribution of the pores, 70% or more of the mesopore volume (the volume of pores having a pore size in the range of 2 to 100 nm) is in a range of ±5 nm of a central pore size of pores in the mesopore region. At least some of the pores in the oxide porous body communicate with each other in the form a three-dimensional network, and the communication channels are formed at random and have a three-dimensional network structure. The oxide porous body does not substantially have a fibrous structure. In addition, the oxide porous body is an oxide porous body obtained by aggregating particles having an aspect ratio of 3 or less, wherein the oxide porous body has pores in gaps between the particles. It is also described that one of the applications of the oxide porous body is a support for an enzyme.

In the case where a porous silica particle is used as a support for immobilizing an enzyme, it is believed that aggregation of the enzyme present in a high density can be prevented and high integration of the enzyme having activity can be realized. Thus, it is expected that, in a reaction system using an enzyme, the enzyme can be made to be present in an amount larger than an amount at which aggregation may be caused in the form of a solution.

The use of an enzyme immobilized on a support has hitherto been conducted. However, most of the intended uses relate to the separation and the reuse of the enzyme. For example, in the known technical fields of decomposition of biomass and production of fiber or food using an enzyme, etc., an operation is often performed using an enzyme in the form of an aqueous solution. In this operation method, an operation of separating the resulting product and the enzyme is necessary, and the separated enzyme is generally disposed of. In order to omit this step of separating an enzyme, research and development on techniques of immobilizing an enzyme, i.e., techniques for using an enzyme in the form of an enzyme immobilized on a support, has been actively conducted.

As a method for immobilizing an enzyme, for example, a method for directly immobilizing an enzyme on a resin bead or the like, microencapsulation by coating with a polymer, and a surface modification method in which the surface of an enzyme protein is stabilized by modification have been proposed. However, in these methods, an enzyme is merely immobilized on the surface of an immobilization support, and the surface area of the support for immobilizing the enzyme is not large. Thus, these methods do not actually aim at a high integration of an enzyme or an improvement in the function of an enzyme due to immobilization.

Immobilization of enzymes for the purpose of reusing the enzymes has also been studied. Also in such a case, these methods do not significantly differ from the methods for the purpose of eliminating the separation step. In many of these methods, an enzyme is simply immobilized on the surface of various types of supports. A method for immobilizing an enzyme on a polymer foam is also employed. In this case, for example, an operation of separating a solution containing a product by compressing the foam is conducted.

The development of a method for immobilizing an enzyme realizes the separation and collection of an enzyme after a reaction and the reuse of the enzyme in a production process using the enzyme. Thus, the development of such a method actually contributes to an increase in the efficiency of the production process. However, a higher integration of an enzyme and an improvement in the function itself of an enzyme due to immobilization of the enzyme have been desired.

PTL 5 discloses an invention related to a hemeprotein-including complex including a silica-based mesoporous body and a hemeprotein contained in pores of the silica-based mesoporous body, wherein (1) the hemeprotein forms a multimer in the pores, and (2) the multimer is adsorbed on the inner walls of the pores of the silica-based mesoporous body as a protein integrated in a high density. According to a finding of this invention, by immobilizing an enzyme, i.e., a protein in pores of a silica-based mesoporous body, thermal stability of the protein and inorganic solvent resistance of the protein are improved.

In general, for example, MCM, FSM, and SBA-type materials are known as a silica-based mesoporous body. These silica-based mesoporous bodies are characterized by having pores with a diameter of 2 to 50 nm. An enzyme, i.e., protein has a size of several nanometers to several tens of nanometers and has substantially the same distribution of the sizes as the distribution of the sizes of the pores of such a silica-based mesoporous body. Therefore, it is believed that in the case where such a silica-based mesoporous body is used as an immobilization support of an enzyme, the enzyme can be immobilized not only on the surface but also in the pores of the silica-based mesoporous body.

Silica-based mesoporous bodies have a surface area effective for immobilizing an enzyme, the surface area being significantly larger than that of porous bodies in the related art. For example, as described in PTL 6, in a complex of a silica-based mesoporous body and a cellulose or hemicellulose-hydrolyzing enzyme, an enzyme can be immobilized in pores thereof and can be integrated.

As a silica diameter mesoporous body for adsorbing a protein such as an enzyme, for example, PTL 7 discloses an invention related to a spherical silica-based mesoporous body having an average particle diameter of 0.01 to 3 $\mu$m and radial pores having a central pore diameter of 1 nm or more, wherein the spherical silica-based mesoporous body is modified with an organic functional group containing a cyano group or a carboxyl group. It is also described that such an organically modified silica diameter mesoporous body exhibits a high adsorption property for a basic dye, a protein, a metal, and the like.

PTL 8 discloses an invention related to a porous spherical silica in which the most frequent pore size is 45 to 70 Å, the average pore size is 50 to 100 Å, and the volume of pores having sizes within a range of $\pm$10% of the most frequent pore size is 0.40 mL/g or more, when the most frequent pore size, the average pore size, and the volume of pores are measured with a mercury porosimeter. PTL 8 also discloses that the porous silica can be produced by baking a porous spherical silica precursor having a BET specific surface area of 400 m$^2$/g or more and a pore volume of 0.9 mL/g or less at a temperature of 600°C or higher. PTL 8 describes that the porous silica is useful as an immobilization support for immobilizing a catalyst, an enzyme, or a microorganism.

PTL 9 discloses an invention related to a complex of a silica-based mesoporous body and a starch hydrolase being a complex of an enzyme that performs hydrolysis of starch and a silica-based mesoporous body on which the starch hydrolase is immobilized, wherein the enzyme immobilized on the silica-based mesoporous body has activity for catalyzing hydrolysis of starch. It is described that an MCM, FSM, or SBA-type silica-based mesoporous body is suitable for the silica-based mesoporous body. It is also described that, regarding characteristics of the silica-based mesoporous body, the pore diameter is preferably in the range of 2 to 50 nm, the total pore volume is preferably in the range of 0.1 to 3.5 mL/g, and the specific surface area is preferably in the range of 200 to 1,500 m$^2$/g.

PTL 10 discloses an invention related to a method for immobilizing an enzyme on a porous silica particle porous body. In this method, an enzyme is immobilized in a pore structural unit of the porous silica particle porous body, the structural unit having an inner diameter 1.2 times or more the diameter of the enzyme and having a structural stability. Subsequently, a network structure of a gelled material is formed by a sol-gel method in an opening portion and/or a gap in the above structural unit to improve stability of the immobilized enzyme.

PTL 11 discloses an invention related to a method for immobilizing an enzyme, the method including stabilizing an internal structure involved in the enzymatic function of an oxidase by, for example, the transformation of an unstable specific amino acid, and immobilizing the oxidase in a pore structural unit of a porous silica particle porous body, the pore structural unit having a predetermined inner diameter and having structural stability to also stabilize the surface of the enzyme.

[Citation List]

[Patent Literature]

[PTL 1] Japanese Unexamined Patent Application Publication No. 10-328558
[PTL 2] Japanese Unexamined Patent Application Publication No. 2006-232594
[PTL 3] Japanese Unexamined Patent Application Publication No. 2009-73681
[PTL 4] Japanese Unexamined Patent Application Publication No. 2001-170500
[PTL 5] Japanese Unexamined Patent Application Publication No. 2007-51076
[PTL 6] Japanese Unexamined Patent Application Publication No. 2009-125006
[PTL 7] Japanese Unexamined Patent Application Publication No. 2008-24567
[PTL 8] Japanese Unexamined Patent Application Publication No. 2007-76941
[PTL 9] Japanese Unexamined Patent Application Publication No. 2009-153448
[PTL 10] Japanese Unexamined Patent Application Publication No. 2001-178457
[PTL 11] Japanese Unexamined Patent Application Publication No. 2002-262863

## SUMMARY

**Problems to Be Solved by the Invention**

In the case where an enzyme is immobilized on a porous silica particle support in the related art, it is necessary to use a porous silica particle having a suitable characteristic (such as a pore size distribution or a pore size) in accordance with the type of enzyme immobilized, the size of the molecule, and/or the isoelectric point of the enzyme. If a porous silica particle that is not suitable for immobilizing a target enzyme is used as a support, the enzyme may be detached from the porous silica particle support, resulting in a difficulty in the repeated use of the enzyme. Even in the case where such a detachment of the enzyme does not occur, the performance of the enzyme ((i) resistance to a reaction temperature with a substrate, (ii) reaction activity to a substrate, (iii) resistance to a solvent used in a reaction with a substrate, and (iv) repetitive usability with regard to a reaction with a substrate) may not be sufficiently exhibited.

In addition, designing and developing a porous silica particle support suitable for immobilization of a specific enzyme each time has been a large burden.

A support for enzyme immobilization according to the present invention has been developed in order to solve the above problems. Specifically, the following objects have been achieved.

(1) An object is to provide a support for enzyme immobilization, the support capable of being applied to various types of enzymes by, for example, an appropriate selection of the particle diameter or the like of silica particles serving as a raw material or by an adjustment at a surface treatment stage without changing the basic structure of the support for each type of enzyme in developing a support for enzyme immobilization, and a method for producing the support. More specifically, in order to provide a support for enzyme immobilization suitable for immobilizing various enzymes having different molecular sizes and different isoelectric points, immobilization of enzymes having various molecular sizes is realized by optimizing the pore size, the pore size distribution, the pore volume, or the pore structure of a porous silica particle, and immobilization of various enzymes is realized by modifying a silanol group on the surface of the porous silica particle.

(2) An object is to provide a method for producing a support for enzyme immobilization and a method for producing an immobilized enzyme (a method for immobilizing an enzyme) which can be applied to industrial mass production.

(3) An object is to design a support for enzyme immobilization in which a change in the three-dimensional structure of an enzyme, the change being a main cause of a decrease in the reaction activity due to immobilization of the enzyme on a support, is suppressed and in which a minute enzyme reaction space for improving the reaction activity of the immobilized enzyme can be ensured in order that reaction activity of the enzyme immobilized on the support for enzyme immobilization to a substrate becomes equal to or higher than the reaction activity before immobilization.

(4) An object is to design a surface treatment for a porous silica particle support for the purpose of suppressing the detachment of an enzyme from a support for enzyme immobilization and to design a structure of a porous silica particle that is not easily degraded or disintegrated even during the repeated use of the immobilized enzyme in order that even when the immobilized enzyme is repeatedly used, the enzyme maintains reaction activity at a practical level and exhibits a sufficient mechanical strength to withstand the repeated use.

(5) An object is to provide an immobilized enzyme having good resistance to a reaction temperature during a reaction between the immobilized enzyme and a substrate or good resistance to a solvent used in the reaction.

**Means for Solving the Problems**

The objects of the present invention are achieved by means of Items [1] to [17] below.

1. A support for protein immobilization, comprising:

porous silica particles each having an interparticle gap structure inside,
wherein the porous silica particles satisfy (1) to (6) below and have a silanol group, an anion-exchange group, or a cation-exchange group on the surfaces thereof:

(1) an average particle diameter (Da) is in the range of 0.5 to 100 $\mu$m;
(2) a specific surface area is in the range of 10 to 250 $m^2$/g;
(3) a pore volume (Pv) is in the range of 0.10 to 0.32 mL/g;
(4) a pore size (Pms) of a peak value in a pore size distribution (X axis: pore size [Ps], Y axis: value obtained by differentiating pore volume with respect to pore size) is in the range of 2 to 200 nm;
(5) a total volume of pores having a pore size in the range of (Pms) $\times$ 0.75 nm to (Pms) $\times$ 1.25 nm is 70% or more of a total volume of all pores; and
(6) a porosity is in the range of 5% to 50%.

2. The support for protein immobilization according to Item 1,
wherein the porous silica particles are composed of spherical aggregates each obtained by aggregating spherical silica fine particles which have an average particle diameter (Db) of 10 to 500 nm, a sphericity in the range of 0.9 to 1, and a coefficient of variation (CV value) of the particle diameter in the range of 2% to 10%, and whose particle diameter distribution exhibits a monodispersion phase.

3. A support for protein immobilization, comprising:

porous silica particles each having an interparticle gap structure inside,
wherein the porous silica particles satisfy (1) to (6) below and have a silanol group, an anion-exchange group, or a cation-exchange group on the surfaces thereof:

(1) an average particle diameter (Da) is in the range of 0.5 to 50 $\mu$m;
(2) a specific surface area is in the range of 10 to 250 $m^2$/g;
(3) a pore volume (Pv) is in the range of 0.10 to 0.32 mL/g;
(4) a pore size (Pms) of a peak value in a pore size distribution (X axis: pore size [Ps], Y axis: value obtained by differentiating pore volume with respect to pore size) is in the range of 2 to 50 nm;
(5) a total volume of pores having a pore size in the range of (Pms) $\times$ 0.75 nm to (Pms) $\times$ 1.25 nm is 80% or more of a total volume of all pores; and

(6) a porosity is in the range of 5% to 50%.

4. The support for protein immobilization according to Item 3,
wherein the porous silica particles are composed of spherical aggregates each obtained by aggregating spherical silica fine particles which have an average particle diameter (Db) of 10 to 50 nm, a sphericity in the range of 0.9 to 1, and a coefficient of variation (CV value) of the particle diameter in the range of 2% to 10% and whose particle diameter distribution exhibits a monodispersion phase.

5. The support for protein immobilization according to any one of Items 1 to 4, wherein the anion-exchange group is a substituent having a structure containing an amino group or a quaternary ammonium group.

6. The support for protein immobilization according to any one of Items 1 to 4, wherein the cation-exchange group is a substituent having a structure containing a group selected from a carboxyl group, a phosphate group, and a sulfoxyl group.

7. The support for protein immobilization according to any one of Items 1 to 6, wherein the porous silica particles are obtained by conducting a surface treatment with a silane coupling agent or an organic acid.

8. The support for protein immobilization according to any one of Items 1 to 7, wherein the porous silica particles are obtained by treating porous silica particles with an amino group-containing silane coupling agent, and further subjecting the porous silica particles to a surface treatment with an organic acid.

9. The support for protein immobilization according to any one of Items 1 to 8, wherein the support is used for immobilizing an enzyme.

10. The support for protein immobilization according to any one of Items 1 to 8, wherein the support is used for immobilizing a multienzyme.

11. An immobilized protein obtained by immobilizing a protein on the support for protein immobilization according to any one of Items 1 to 8.

12. The immobilized protein according to Item 11, wherein the protein is an enzyme.

13. The immobilized protein according to Item 12, wherein the enzyme is a racemase.

14. The immobilized protein according to Item 12, wherein the enzyme is deoxyriboaldolase.

15. A method for producing an immobilized protein, comprising a step of allowing a protein to adsorb onto the support for protein immobilization according to any one of Items 1 to 8.

16. The method according to Item 15, wherein the protein is an enzyme.

17. The method for producing an immobilized protein according to Item 15 or 16, wherein the protein is allowed to adsorb onto the support for protein immobilization in a buffer solution in the range of 4°C to 25°C.

The support for protein immobilization according to the present invention includes porous silica particles each of which has an interparticle gap structure inside, in which the uniformity of the pore size is high, and which have a silanol group, an anion-exchange group, or a cation-exchange group on the surfaces thereof. In particular, since the uniformity of the pore size is high, by selecting a support for protein immobilization, the support having a pore size suitable for the molecular size of a protein, in particular, an enzyme, the protein is uniformly immobilized on the support for enzyme immobilization. In addition, by selecting the type of substituent on the surface of the support for protein immobilization in accordance with the type of protein such as an enzyme, the protein is stably immobilized on the support for enzyme immobilization.

The support for protein immobilization according to the present invention is usually composed of a spherical aggregate of spherical silica fine particles. The particle diameter distribution of the spherical silica fine particles exhibits a monodispersion phase, thereby achieving the uniformity of the pore size of the support for protein immobilization according to the present invention. The reason why the support for protein immobilization according to the present invention is not easily degraded or disintegrated is that the support for protein immobilization has a stable structure of an aggregate composed of uniform spherical silica fine particles. Furthermore, the support for protein immobilization according to the present invention can be used for immobilizing proteins, in particular, enzymes having various molecular sizes by simply selecting the particle diameter of the spherical silica fine particles in the stage of the production of the support.

In a preferred embodiment of the support for protein immobilization according to the present invention, the porous silica particles constituting the support for protein immobilization have an anion-exchange group or a cation-exchange group on the surfaces thereof. In particular, the porous silica particles may have, as the anion-exchange group, a substituent having a structure containing an amino group or a quaternary ammonium group or may have, as the cation-exchange group, a substituent having a structure containing a group selected from a carboxyl group, a phosphate group, and a sulfoxyl group. This is advantageous in that a protein, in particular, an enzyme can be immobilized through a strong bond such as a covalent bond. Such a substituent is typically formed by treating porous silica particles with a silane coupling agent to modify a silanol group on the surfaces of the particles. According to the support for protein immobilization of the present invention, in the stage of the production of the support, by conducting a selection of the substituent introduced by a chemical agent such as a silane coupling agent in addition to a selection of the particle diameter of the spherical silica fine particles, an optimum support for protein immobilization can be produced in accordance with the type of protein.

The immobilized protein according to the present invention is structurally stable and has a highly uniform pore size distribution because a support for protein immobilization used for immobilizing a protein is composed of a spherical aggregate of highly uniform spherical silica fine particles. In addition, since the support for protein immobilization has, on the surface thereof, a substituent according to a surface charge of a protein, the protein adsorbed on the support for protein immobilization can stably exist. With the features described above, the immobilized protein according to the present invention can be applied to immobilization of various proteins by appropriately selecting the pore size and the type of substituent of the surface within the design range of the immobilized protein. Furthermore, because of the stability of the structure of the immobilized protein, the immobilized protein according to the present invention is not easily degraded or disintegrated even during repeated use and is not easily affected by the influence of the temperature or the influence of a solvent in a reaction with a substrate.

The immobilized protein according to the present invention has the excellent features described above. Thus, an immobilized enzyme obtained by using an enzyme as the protein can exhibit reaction activity equal to or higher than that of the enzyme before immobilization.

The method for producing an immobilized enzyme according to the present invention has a high practical utility, and conditions to be controlled in the method are also not strict. Thus, the method is also suitable for mass production.

**Effects of the Invention**

A support for protein immobilization according to a first aspect of the present invention includes porous silica particles each of which has an interparticle gap structure inside, in which the uniformity of the pore size is high, and which have a silanol group, an anion-exchange group, or a cation-exchange group on the surfaces thereof. In particular, since the uniformity of the pore size is high, a protein can be uniformly immobilized on the support for protein immobilization by selecting a support for protein immobilization having a pore size suitable for the molecular size of the protein, in particular, an enzyme. Furthermore, the protein can be stably immobilized on the support for protein immobilization by selecting the type of substituent on the surface of the support for protein immobilization in accordance with the type of protein. In the case where this support for protein immobilization is applied to the development of a novel immobilized protein, in particular, a novel immobilized enzyme, the following advantages are achieved.

(1) Various enzymes can be immobilized on the support.

(2) A method for immobilizing an enzyme is easily performed.

(3) An enzyme can be immobilized while maintaining reaction activity equal to or higher than the reaction activity of a free enzyme.

(4) Since the support for enzyme immobilization is obtained by using a porous silica particle as a raw material and has a stable structure, the resulting immobilized enzyme has a high mechanical strength and can be easily repeatedly used.

(5) Industrial mass production of an immobilized enzyme can be realized.

(6) The development period regarding immobilization of a target enzyme to be developed can be shortened, and thus the development can be easily led to industrialization.

(7) Since an enzyme has high activity even after immobilization, the loss of the enzyme due to immobilization can be suppressed.

(8) Any of an acidic enzyme, a basic enzyme, a neutral enzyme, and a hydrophobic enzyme can be immobilized by, for example, modifying the surface of the porous silica particle with a hydroxyl group, an amino group, a carboxyl group, a phenylamino group, or the like.

An immobilized protein according to a second aspect of the present invention is an immobilized protein obtained by immobilizing a protein on the above-described support for protein immobilization. This immobilized protein achieves, for example, the following advantages.

(a) When the immobilized protein is used as an immobilized enzyme, the immobilized enzyme can exhibit reaction activity equal to or higher than the reaction activity of a free enzyme.

(b) The immobilized protein has a high mechanical strength and can be easily repeatedly used.

(c) Industrial mass production can be realized.

(d) When the immobilized protein is used as an immobilized enzyme, the immobilized enzyme has high activity and thus the loss of the enzyme due to immobilization can be suppressed.

(e) When the immobilized protein is used as an immobilized enzyme, the immobilized enzyme is excellent in terms of heat resistance and solvent resistance during a reaction between the immobilized enzyme and a substrate.

(f) In particular, an immobilized protein obtained by immobilizing an enzyme selected from a racemase, glucoamylase, amylase, and laccase is excellent in terms of heat resistance.

(g) In particular, an immobilized protein obtained by immobilizing DERA is excellent in terms of substrate tolerance and a shift of equilibrium.

**BRIEF DESCRIPTION OF DRAWINGS**

These and other objects and features of the present invention will become clearer from the following description of the preferred embodiments given with reference to the attached drawings, wherein:

FIG. 1 is a graph illustrating specific activity of racemase immobilized on various types of supports for protein immobilization;
FIG. 2 is a graph illustrating a change in specific activity of various types of immobilized racemase when the immobilized racemase was repeatedly used;
FIG. 3 is a graph illustrating a change in specific activity of various types of immobilized racemase when the immobilized racemase was repeatedly used;
FIG. 4 is a schematic view illustrating a reaction with the mediation of deoxyriboaldolase (DERA);
FIG. 5 is a schematic view illustrating a lactone synthesis reaction by a tandem aldol reaction;
FIG. 6 is a graph illustrating specific activity of DERA immobilized on various types of supports for protein immobilization in a forward reaction;
FIG. 7 is a graph illustrating the comparison of a change in specific activity in the forward reaction in acetaldehyde with time between the case where immobilized DERA was used and the case where free DERA was used;
FIG. 8 is a graph illustrating the comparison of a change in the amount of reverse reaction product in acetaldehyde with time between the case where immobilized DERA was used and the case where free DERA was used;
FIG. 9 is a graph illustrating the comparison of the amount of lactone produced between the case where immobilized DERA was used and the case where free DERA was used;
FIG. 10 is a graph illustrating specific activity of nitrile hydrase (NHase) immobilized on various types of supports for protein immobilization;
FIG. 11 is a graph illustrating the distribution between the isoelectric point and the molecular weight of various enzymes including immobilized enzymes of the present invention;and
FIG. 12 is a graph illustrating pore size distributions of various supports for protein immobilization. (The curves joining the circular symbols indicate pore size distributions measured by a nitrogen adsorption method, and the curves joining the triangular symbols indicate pore size distributions measured by a mercury penetration method. The values described near peaks of each of the curves represent the pore sizes of the support for protein immobilization.

**DESCRIPTION OF EMBODIMENTS**

In the present invention, an adsorption phenomenon of a protein on a porous silica particle composed of an aggregate of fine particles and having mesopores or a porous silica particle subjected to a treatment of providing a particular functional group on the surface of the above porous silica particle using a silane coupling agent or the like is utilized as a method for immobilizing the protein. Thus, the protein is stably immobilized in the pores or on the surface of the porous silica particle to sufficiently exhibit the function of the immobilized protein.

In the present invention, an enzyme is preferably selected as the protein immobilized on the porous silica particle. Accordingly, the present invention includes a support for enzyme immobilization or an immobilized enzyme.

1. Support for protein immobilization

A support for protein immobilization according to the present invention includes specific porous silica particles each having an interparticle gap structure inside. The porous silica particles used in the present invention satisfy (1) to (6) below and have a silanol group, an anion-exchange group, or a cation-exchange group on the surfaces thereof:

(1) an average particle diameter (Da) is in the range of 0.5 to 100 $\mu$m;
(2) a specific surface area is in the range of 10 to 250 $m^2$/g;
(3) a pore volume (Pv) is in the range of 0.10 to 0.32 mL/g;
(4) a pore size (Pms) of a peak value in a pore size distribution (X axis: pore size [Ps], Y axis: value obtained by differentiating pore volume with respect to pore size) is in the range of 2 to 200 nm;
(5) a total volume of pores having a pore size in the range of (Pms) $\times$ 0.75 nm to (Pms) $\times$ 1.25 nm is 70% or more of a total volume of all pores; and
(6) a porosity is in the range of 5% to 50%.

The support for protein immobilization according to the present invention is used as a support for immobilizing a protein in an immobilized protein described below. The support for protein immobilization according to the present invention is used as a support for immobilizing an enzyme in a preferred embodiment. Accordingly, the support for protein immobilization according to the present invention may also be particularly referred to as "support for enzyme immobilization" for the purpose of emphasizing this aspect.

1-1. Porous silica particles

Porous silica particles in the present invention are porous silica particles each of which has an interparticle gap structure inside, which satisfy the following conditions:

1) an average particle diameter (Da) of the porous silica particles is in the range of 0.5 to 100 $\mu$m;
2) a specific surface area is in the range of 10 to 250 m$^2$/g;
3) a pore volume is in the range of 0.10 to 0.32 mL/g;
4) a pore size (Pms) of a peak value in a pore size distribution (X axis: pore size [Ps], Y axis: value obtained by differentiating pore volume with respect to pore size) is in the range of 2 to 200 nm;
5) a total volume of pores having a pore size in the range of (Pms) $\times$ 0.75 nm to (Pms) $\times$ 1.25 nm is 70% or more of a total volume of all pores; and
6) a porosity is in the range of 5% to 50%, and

which have a silanol group, an anion-exchange group, or a cation-exchange group on the surfaces thereof.

In the porous silica particles used in the present invention, the interparticle gap structure is usually formed by self accumulation and/or self assembly of particles. The porous silica particles used in the present invention are composed of spherical aggregates each obtained by aggregating spherical silica fine particles usually having an average particle diameter of 10 to 500 nm and a sphericity in the range of 0.9 to 1, preferably having an average particle diameter of 10 to 50 nm and a sphericity in the range of 0.9 to 1, and having a high uniformity of the particle diameter.

Regarding the interparticle gap structure of each of the porous silica particles according to the present invention, in particular, a pore size (Pms) of a peak value in a pore size distribution (X axis: pore size, Y axis: value obtained by differentiating pore volume with respect to pore size) is in the range of 2 to 200 nm, preferably in the range of 2 to 50 nm, and a total volume of pores having a pore size in the range of (Pms) x 0.75 nm to (Pms) x 1.25 nm is 70% or more, and preferably 80% or more of a total volume of all pores. With this configuration, the tendency in which the catalyst or the like is sufficiently dispersed and supported on the porous silica particles is accelerated. In addition, reaction selectivity can be imparted to the catalyst support on which the catalyst is supported. It is believed that this is because the particles that form the pores have a spherical shape and uniform particle diameter, and thus the formed pores also have a uniform pore size and are present in a dispersed state.

The average particle diameter (Da) of the porous silica particles according to the present invention is preferably in the range of 0.5 to 100 $\mu$m, and more preferably in the range of 0.5 to 50 $\mu$m. According to a production method of the present invention described below, when the average particle diameter is within this range, spherical, and uniform porous silica particles can be obtained. Porous silica particles having an average particle diameter of less than 0.5 $\mu$m are not easily prepared by the production method of the present invention. An average particle diameter exceeding 50 $\mu$m, in particular, exceeding 100 $\mu$m is not preferable because particles having an irregular shape tend to be generated by the production method of the present invention. It is recommended that the average particle diameter of the porous silica particles be preferably in the range of 5 to 50 $\mu$m, and particularly preferably in the range of 5 to 30 $\mu$m. The average particle diameter of the porous silica particles is measured by a centrifugal sedimentation method. A specific measuring method is described in 1. (B) "Method for measuring average particle diameter by centrifugal sedimentation method" in [Methods for measuring physical properties] of Examples.

The porous silica particles according to the present invention preferably have a specific surface area in the range of 10 to 250 m$^2$/g. In the case where porous silica particles having a specific surface area of less than 30 m$^2$/g, in particular, less than 10 m$^2$/g are used as a support, the support is used in a large amount in many cases, and this is economically disadvantageous. A specific surface area exceeding 250 m$^2$/g is not preferable because, for example, readsorption of a reaction product occurs, which may result in a decrease in the reaction efficiency, and the strength of the spherical aggregates becomes insufficient. It is recommended that the specific surface area be more preferably in the range of 30 to 250 m$^2$/g.

The porous silica particles according to the present invention have a pore volume in the range of 0.10 to 0.32 mL/g. When porous silica particles having a pore volume of less than 0.10 mL/g are used as a support, the amount of catalyst supported, the catalyst being a metal fine particle or the like, is decreased. Accordingly, the support is used in a large amount in many cases, and this is economically disadvantageous. When the pore volume exceeds 0.32 mL/g, the strength of the spherical aggregates may become insufficient. The pore volume is preferably in the range of 0.10 to 0.25 mL/g, and more preferably in the range of 0.12 to 0.20 mL/g.

The pore volume can be determined by a constant-volume gas adsorption method using nitrogen. The pore distribution and the pore size (peak value) can be determined by the Barrett-Joyner-Halenda (BJH) method. Alternatively, a mercury penetration method may also be used as a method for measuring these values.

In the porous silica particles according to the present invention, regarding the interparticle gap structure of each of the porous silica particles according to the present invention, in particular, a pore size (Pms) of a peak value in a pore size distribution (X axis: pore size [Ps], Y axis: value obtained by differentiating pore volume with respect to pore size) is in the range of 2 to 200 nm, and a total volume of pores having a pore size in the range of (Pms) x 0.75 nm to (Pms) x 1.25 nm is 70% or more of a total volume of all pores.

When the pore size (Pms) is less than 2 nm, it is not easy to ensure a necessary pore volume. When the pore size (Pms) exceeds 25 nm, in particular, exceeds 200 nm, a decrease in the particle strength may cause a practical problem. It is recommended that the pore size (Pms) be preferably in the range of 2 to 50 nm, and more preferably in the range of 3 to 15 nm.

When the total volume of pores having a pore size in the range of (Pms) x 0.75 nm to (Pms) x 1.25 nm is less than 80%, in particular, less then 70% of the total volume of all pores, the pore size distribution is not uniform, and a stress is concentrated on relatively large pores. Consequently, a problem of, for example, a decrease in the strength tends to occur in terms of practical use. It is recommended that the above total volumes of the pores relative to the total volume of all pores be 80% or more, and more preferably 85% or more.

The porous silica particles used in the present invention preferably have a porosity in the range of 5% to 50%. The porous silica particles of the present invention can exhibit an excellent particle fracture strength even when the porous silica particles have a porosity within this range. When the porosity is less than 5%, the amount of substance that can be supported is very small, and this is not suitable for practical use. A porosity exceeding 50% is not preferable because the strength of the particles may not be maintained. It is recommended that the porosity be preferably in the range of 10% to 30%.

Spherical aggregate

The porous silica particles according to the present invention are composed of spherical aggregates of spherical silica fine particles as described above. The average particle diameter (Db) of the spherical silica fine particles is preferably in the range of 10 to 500 nm. When the average particle diameter is less than 10 nm, the pore volume formed by gaps between the inorganic silica fine particles decreases because the particle diameter is excessively small. Thus, the practical utility is decreased when such spherical silica fine particles are used as particles for a support. When the average particle diameter exceeds 50 nm, in particular, exceeds 500 nm, although the pore volume is increased, the bonding strength between the fine particles is weak and it is difficult to obtain aggregates of the spherical silica fine particles. The average particle diameter of the spherical silica fine particles is more preferably in the range of 10 to 300 nm, still more preferably in the range of 10 to 50 nm, and particularly preferably in the range of 10 to 48 nm.

In the present application, the average particle diameter of the spherical silica fine particles refers to an average particle diameter measured by a dynamic light scattering method or an average particle diameter measured by an image analysis method.

In the cases of "spherical silica fine particles (a)" and "spherical silica fine particles (b)" described below, the term "average particle diameter" means the same as the above. Regarding the method for measuring an average particle diameter by an image analysis method, the measurement was conducted by the method for measuring an average particle diameter described in "5. Measurement of particle size distribution" in [Methods for measuring physical properties] of Examples.

The spherical silica fine particles do not contain particles having an irregular shape such as a rod shape, a comma shape, an elongated shape, a chain-like shape, or an oval shape, and have a high sphericity. In the present invention, the term "spherical shape" refers to a shape having a sphericity in the range of 0.90 to 1.00. Herein, the term "sphericity" refers to an average of a ratio (DS/DL) of a maximum diameter (DL) to a short diameter (DS) orthogonal to the maximum diameter of 50 arbitrary particles in a photographic projection image obtained by taking a photograph with a transmission electron microscope. In the case where the sphericity is less than 0.90, the fine particles are not spherical and may contain particles corresponding to the irregular particles described above.

The porous silica particles of the present invention obtained by using, as the spherical silica fine particles, spherical silica fine particles having a sphericity of 0.90 to 1.00 can exhibit an excellent particle fracture strength. In particular, making the sphericity of the spherical silica fine particles uniform to a level of 0.90 or more significantly affects the strength of the porous silica particles.

As the spherical silica fine particles, it is possible to use, for example, silica fine particles of an oxide sol disclosed in Japanese Unexamined Patent Application Publication No. 5-132309, organic group-containing composite silica fine particles disclosed in Japanese Unexamined Patent Application Publication No. 10-454043, or composite silica fine particles having gaps inside thereof disclosed in Japanese Unexamined Patent Application Publication No. 7-133105. In the case where the sphericity of the silica fine particles is less than the above value, the sphericity is adjusted to a range of 0.90 to 1.00 by conducting a so-called hydrothermal treatment, and the particles may then be used as the spherical silica fine particles. The hydrothermal treatment is conducted, for example, under the conditions of a temperature of 100°C to 200°C for 1 to 24 hours. It is also recommended to use an autoclave in the hydrothermal treatment.

In the case where the particle diameter distribution of the spherical silica fine particles is a monodispersion, it is recommended that the coefficient of variation (CV value) of the particle diameter of the spherical silica fine particles be preferably in the range of 2% to 10%. A coefficient of variation (CV value) of the particle diameter of less than 2% is more desirable for the present invention. However, it is not easy to obtain spherical silica fine particles having a particle diameter distribution at such a level. In the case where the coefficient of variation of the particle diameter exceeds 10%, the degree of monodispersion decreases, and thus the advantage of the present invention decreases. It is recommended that the coefficient of variation of the particles be preferably in the range of 2% to 7%.

Known methods can be employed as the method for producing spherical aggregates of such spherical silica fine particles. Examples of the method include a microcapsule method, an emulsification method, an oil method, and a spraying method. Among these methods, the method for producing spherical fine particle powder disclosed in, for example, Japanese Examined Patent Application Publication Nos. 3-43201 and 2-61406 and filed by the applicant of the present application is advantageous in that spherical, inorganic silica fine particle aggregates are obtained even in the case where starting inorganic silica fine particles are not spherical, and is also economically advantageous because the production process is not complicated. This preferable production method is described below.

Functional group

The porous silica particles used in the present invention have a silanol group, an anion-exchange group, or a cation-exchange group as a functional group on the surfaces thereof.

Specifically, in an embodiment of the support for protein immobilization of the present invention, porous silica particles having a silanol group are used as the porous silica particles. Here, since porous silica particles have a silanol group on the surfaces thereof, the porous silica particles are included in the support for protein immobilization according to the present invention.

In another embodiment, porous silica particles having an anion-exchange group or a cation-exchange group are used as the porous silica particles. In the present invention, the phrase "having an anion-exchange group" or "having a cation-exchange group" refers to a case where functional groups on the surfaces of the porous silica particles include an anion-exchange group or a cation-exchange group, and all the silanol groups need not be modified with an anion-exchange group or a cation-exchange group.

Accordingly, examples of embodiments of porous silica particles in the support for protein immobilization of the present invention mainly include (i) porous silica particles that contain a silanol group but that do not contain a anion-exchange group or a cation-exchange group, (ii) porous silica particles that contain an anion-exchange group and that do not contain a cation-exchange group, and (iii) porous silica particles that contain a cation-exchange group and that do not contain an anion-exchange group. In some cases, examples thereof may include (iv) porous silica particles that contain both an anion-exchange group and a cation-exchange group.

In the present invention, the term "anion-exchange group" refers to a group that can adsorb an anion or a group that reacts with an acid and converted to an anion to exhibit hydrophilicity, and refers to a cationic group or a basic group. Examples of such a group include groups having a structure containing an amino group and groups having a structure containing a quaternary ammonium group. Examples of the groups having a structure containing an amino group include, but are not limited to, groups containing a primary amino group, the groups being typified by $-CH_2CH_2CH_2NH_2$, $-CH_2CH_2CH_2CH_2NH_2$, and other aminoalkyl groups; groups containing a secondary amino group, such as a phenylamino group; and groups containing a tertiary amino group, such as $-CH_2N(CH_3)_2$, $-CH_2CH_2N(CH_3)_2$, $-CH_2CH_2N(CH_2CH_2)_2$, and $-CH_2CH_2CH_2N(C_H2C_H2)_2$. Examples of the groups having a structure containing a quaternary ammonium group include, but are not limited to, $-CH_2N^+(CH_3)_3$, $-CH_2CH_2N^+(CH_3)_3$, $-CH_2CH_2N^+(CH_2CH_2)_3$, $-CH_2CH_2CH_2N^+(CH_2CH_2)_3$, $-CH_2CH_2N^+(CH_2CH_3)_2CH_2CH(OH)CH_3$, and $-N^+(CH_3)_3$.

On the other hand, the term "cation-exchange group" refers to a group that can adsorb a cation or a group that reacts with a base and converted to a cation to exhibit hydrophilicity, and refers to an anionic group or an acidic group. Here, although a silanol group can also adsorb a cation, the term "cation-exchange group" in the present invention refers to a cation-exchange group other than a silanol group in a strict sense. Examples of such a cation-exchange group include groups having a structure containing a group selected from a carboxyl group, a phosphate group, and a sulfoxyl group. Examples of the groups having a structure containing a carboxyl group include carboxy alkyl groups such as $-CH_2COOH$, $-CH_2CH_2COOH$, $-CH_2CH_2CH_2COOH$, and $-CH_2CH_2CH_2CH_2COOH$. An example of the phosphate group is $-PO_4H_2$. Examples of the groups having a structure containing a sulfoxyl group include sulfoalkyl groups such as $-CH_2CH_2SO_3H$ and $-CH_2CH_2CH_2SO_3H$. However, examples of the above groups are not limited thereto.

In the support for protein immobilization of the present invention, these anion-exchange group and cation-exchange group are usually introduced on the surfaces of the porous silica particles through bonding functional groups to the porous silica particles. In a typical case, the anion-exchange group and the cation-exchange group are bonded to the surfaces of the porous silica particles by treating silanol groups on the surfaces of the porous silica particles with a silane coupling agent or an organic acid. In the case where the treatment is conducted with a silane coupling agent, a desired anion-exchange group and cation-exchange group are introduced by a hydrolysis reaction between the silanol groups on the surfaces of the porous silica particles and the silane coupling agent. In such a case, the anion-exchange group and the cation-exchange group are bonded to the surfaces of the porous silica particles through silicon atoms of the silane coupling agent.

In this description, the terms "anion-exchange group" and "cation-exchange group" may be used to represent a partial molecular structure including a bonding functional group. For example, the term "group having a structure containing an amino group" may be used to represent a group including a bonding functional group derived from a silyl coupling agent, e.g., $-Si-CH_2CH_2N(CH_3)_2$.

The porous silica particles used in the present invention preferably have a silanol group, an anion-exchange group, or a cation-exchange group as a functional group on the surfaces thereof. Besides these groups, hydrophobic organic groups may also be preferably used. Examples of the hydrophobic organic groups include, but are not limited to, a methyl group and a $\gamma$-methacryloxypropyl group.

Surface treatment

Regarding the porous silica particles used in the present invention, the spherical aggregates each obtained by aggregating the spherical silica fine particles may further be subjected to a surface treatment according to need in addition to the above introduction of the "functional group". The surface treatment is performed within a range where the pore volume and the pore size can be maintained in the specified ranges described above. This surface treatment can increase the strength of the particles. In the case where the porous silica particles are used as a support, the surface treatment has effects of increasing the affinity with the substance to be supported, and increasing a supporting force.

In the case where a surface treatment is performed by adding an acid or an alkali and an organosilicon compound represented by a general formula below and/or a partial hydrolysate thereof to spherical silica fine particles, a silica-based coating layer having an organic functional group is formed.

General formula: $R_nSi(OR')_{4-n}$

[In the formula, R and R' each represent a hydrocarbon group selected from an alkyl group having 1 to 18 carbon atoms, an aryl group having 1 to 18 carbon atoms, a vinyl group, and an acrylic group, and n represents an integer of 0, 1, 2, or 3.]

1-2. Method for producing porous silica particles

The method for producing porous silica particles used in the present invention is not particularly limited as long as the objects of the present invention are achieved and desired operations and advantages are obtained. However, the porous silica particles used in the present invention are preferably produced as spherical porous particles, and are usually produced by the method described below in order to obtain such spherical porous particles.

The method for producing such porous silica particles include steps of (A), (B), and (C) described below as a method for producing spherical porous particles.

(A) Centrifugal separation process

A dispersion liquid of spherical silica fine particles having an average particle diameter of 10 to 500 nm, and preferably 10 to 50 nm is prepared, and coarse particles are then separated by conducting a centrifugal separation process to adjust the coefficient of variation (CV value) of the particle diameter in the range of 2% to 10%, thus preparing a spherical silica fine particle dispersion liquid whose particle diameter distribution exhibits a monodispersion phase.

Regarding the conditions for the centrifugal separation process, in general, it is recommended that the solid concentration of the spherical silica fine particle dispersion liquid be 1% to 50% by mass, and the centrifugal force be in the range of 500 to 20,000 x g. Herein, the unit "x g" refers to a relative centrifugal force (RCF) that is represented by a ratio to the gravitational acceleration of the earth, and may also be denoted by "G".

In the case where a dispersion liquid of spherical silica fine particles which have a coefficient of variation (CV value) of the particle diameter in the range of 2% to 10% and whose particle diameter distribution exhibits a monodispersion phase is available as a raw material and used, this centrifugal separation process can be omitted.

(B) Preparation of spherical silica fine particle aggregate

A spray liquid containing a spherical silica fine particle dispersion liquid is sprayed in an air flow to prepare spherical silica fine particle aggregates. Water or an organic solvent is used as a solvent of the spherical silica fine particle dispersion liquid. For example, a monohydric alcohol such as ethanol, propanol, or butanol or a polyhydric alcohol such as ethylene glycol can be used as the organic solvent.

The spray liquid may optionally contain a silicic acid liquid in addition to the spherical silica fine particle dispersion liquid. The addition of the silicic acid liquid to the spray liquid containing the spherical silica fine particle dispersion liquid has an effect of increasing the strength of the particles. Regarding the amount of silicic acid liquid added, a ratio [mass of spherical silica fine particle]/silicic acid liquid (on a silica basis) is preferably 1.3 or more. When the ratio is less than 1.3, the proportion of silica derived from the silicic acid liquid is excessive, and the tendency of decreasing the porosity increases.

The concentration of the spray liquid is preferably in the range of 2% to 60% by weight, and particularly preferably in the range of 4% to 50% by weight in terms of solid content. When the solid concentration of the spray liquid is less than 2% by weight, it is difficult to obtain aggregates. When the concentration of the spray liquid exceeds 60% by weight, the spray liquid becomes unstable, and it becomes difficult to obtain spherical aggregates. In addition, the spray drying described below cannot be continuously conducted, resulting in a decrease in the yield of the aggregates.

The method of spray drying of the spray liquid is not particularly limited as long as the aggregates described above can be obtained. Any known method such as a rotating-disk method, a pressure nozzle method, or a two-fluid nozzle method can be employed. In particular, the two-fluid nozzle method disclosed in Japanese Examined Patent Application Publication No. 2-61406 is preferable because spherical silica fine particle aggregates having a uniform particle diameter distribution can be obtained and the average particle diameter can be easily controlled.

Regarding the drying temperature in the spray drying, in the case where a spray dryer is used, for example, an inlet temperature of the spray dryer is preferably 100°C to 300°C and an outlet temperature of the spray dryer is preferably 40°C to 100°C, though the drying temperature depends on the concentration of the spherical silica fine particle dispersion liquid, the processing speed, and the like. More preferably, it is recommended that the inlet temperature be in the range of 210°C to 250°C and the outlet temperature be in the range of 50°C to 55°C.

The spray speed is, for example, in the range of 0.1 to 3 L/hour, though it depends on the shape or the size of a spray device.

(C) Heat treatment of spherical silica fine particle aggregates

The spherical silica fine particle aggregates obtained in step (B) are heat-treated in a temperature range of 150°C to 600°C in order to increase the bonding strength between spherical silica fine particles or between the particle and a gel component. When the heat-treatment temperature is lower than 150°C, the effect of improving the bonding strength may not be obtained. On the other hand, a heat-treatment temperature exceeding 600°C is also not preferable because the spherical silica fine particle aggregates may be shrunk and the size of gaps between the finally obtained spherical porous particles may be decreased.

After steps (A), (B), and (C) described above, processes of steps (D), (E), and (F) described below may be conducted according to need.

(D) Preparation of spherical silica fine particle aggregate dispersion liquid

The spherical silica fine particle aggregates obtained in step (C) are left to cool or cooled to room temperature to 40°C, and dispersed in water and/or an organic solvent to prepare a dispersion liquid thereof. For example, a monohydric alcohol such as ethanol, propanol, or butanol or a polyhydric alcohol such as ethylene glycol can be used as the organic solvent. The concentration of the dispersion liquid is preferably in the range of 0.1% to 40% by weight, and particularly preferably 0.5% to 20% by weight in terms of an oxide of the spherical silica fine particle aggregate. On the other hand, a concentration exceeding 40% by weight is not preferable because the aggregates tend to aggregate to each other in step (D).

(E) Surface treatment

A surface treatment of the outer surfaces of the spherical silica fine particle aggregates is conducted by adding the following i) or ii) to the aggregate dispersion liquid obtained in step (D).

    i) An acid or an alkali

ii) An acid or an alkali, and an organosilicon compound represented by a general formula below and/or a partial hydrolysate thereof:

General formula: $R_nSi(OR')_{4-n}$

[wherein R and R' each represent a hydrocarbon group which may be substituted with a group that does not contain an ion-exchange group and which is selected from an alkyl group having 1 to 18 carbon atoms, an aryl group having 1 to 18 carbon atoms, a vinyl group, and an acrylic group, and n represents an integer of 0, 1, 2, or 3.]

As for the acid or the alkali in the case of i) above, an aqueous solution of an acid or an alkali is usually used. Here, as the "acid" used in i) above, unlike an organic acid used in the production of a support for protein immobilization described below, an acid that does not introduce a cation-exchange group on the outer surfaces of the spherical silica fine particle aggregates is used. Examples of the acid and the alkali include, but are not particularly limited to, an aqueous hydrochloric acid solution, an aqueous boric acid solution, and an aqueous ammonium solution.

The acid or the alkali in the case of ii) above is defined as in the case of i). Here, as the "organosilicon compound" used in ii) above, unlike a silane coupling agent used in the production of a support for protein immobilization described below, an organosilicon compound that does not introduce an anion-exchange group or a cation-exchange group on the outer surfaces of the spherical silica fine particle aggregates is used. Specific examples of the organosilicon compound represented by the above general formula include tetramethoxysilane, tetraethoxysilane, tetraisopropoxysilane, methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyltris(β-methoxyethoxy)silane, 3,3,3-trifluoropropyltrimethoxysilane, methyl-3,3,3-trifluoropropyldimethoxysilane, β-(3,4-epoxycyclohexyl)ethyltrimethoxysilane, γ-glycidoxytripropyltrimethoxysilane, γ-glycidoxypropylmethyldiethoxysilane, γ-glycidoxypropyltriethoxysilane, γ-methacryloxypropylmethyldimethoxysilane, γ-methacryloxypropyltrimethoxysilane, γ-methacryloxypropylmethyldiethoxysilane, γ-methacryloxypropyltriethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, and diethylsilane.

The acid or the alkali added together with the organosilicon compound and/or a partial hydrolysate thereof also functions as a catalyst for hydrolysis. However, a catalyst for hydrolysis may be added according to need. In the case where a basic catalyst such as a hydroxide of an alkali metal, aqueous ammonia, or an amine is used as the hydrolysis catalyst, the basic catalyst may be removed after hydrolysis, and the resulting liquid may be used as an acidic solution. In the case where a mixture for hydrolysis is prepared by using an acid catalyst such as an organic acid or an inorganic acid, it is preferable to remove the acid catalyst after hydrolysis by ion exchange or the like. The prepared mixture for hydrolysis of the organosilicon compound is preferably used in the form of an aqueous solution. Herein, the term "aqueous solution" refers to a state where the mixture for hydrolysis is not present in a white turbid state as a gel but in a state having transparency.

Organosilicon compounds where n in the general formula is 0 can be used without further treatment. However, compounds where n is 1 to 3 have poor hydrophilicity, and thus are preferably hydrolyzed in advance so as to be uniformly mixed in a reaction system. Any method known as a method for hydrolyzing such an organosilicon compound can be employed for the hydrolysis.

A precursor metal salt of an inorganic oxide other than the above-described oxide may be added together with the organosilicon compound and/or a partial hydrolysate thereof or a silicic acid liquid to form an oxide-based layer composed of the oxide and the inorganic oxide other than the oxide. As the material of the inorganic oxide other than the oxide, an alkali-soluble inorganic compound is preferably used. Examples thereof include alkali metal salts, alkaline earth metal salts, ammonium salts, and quaternary ammonium salts of an oxoacid of the metal or nonmetal described above.

(F) Heat treatment

Furthermore, spherical silica fine particle aggregates are separated from the spherical silica fine particle aggregate dispersion liquid obtained in step (E), dried, and then heat-treated under atmospheric pressure or reduced pressure at 100°C to 300°C to obtain porous silica particles.

1-3. Method for producing support for protein immobilization

Regarding a support for protein immobilization, the support being composed of a porous silica particle having a silanol group on the surface thereof, a porous silica particle produced by the method described in 1-2. above can be used without further treatment. Among porous silica particles produced by the above method, a porous silica particle obtained through steps (A), (B), and (C) described above is particularly preferable. Such a porous silica particle originally has a silanol group on the surface thereof. In other words, this porous silica particle can be used as a support for protein immobilization of the present invention for immobilizing a protein such as an enzyme.

Meanwhile, a support for protein immobilization, the support being composed of a porous silica particle having an anion-exchange group or a cation-exchange group on the surface thereof, is produced by introducing an anion-exchange group or a cation-exchange group to the porous silica particle produced by the method described in 1-2. above. Preferably, the porous silica particle having an anion-exchange group or a cation-exchange group on the surface thereof is obtained by treating a porous silica particle having a silanol group on the surface thereof and obtained through steps (A), (B), and (C) described above with a silane coupling agent or an organic acid. As the silane coupling agent or the organic acid used in this treatment, a silane coupling agent or organic acid having an anion-exchange group or a cation-exchange group is used.

A support for protein immobilization, the support being composed of a porous silica particle having a hydrophobic organic group on the surface thereof, is obtained by treating a porous silica particle obtained through steps (A), (B), and (C) described above with a silane coupling agent (having an hydrophobic organic group).

Examples of the silane coupling agent used in the method for producing a support for protein immobilization according to the present invention include silane coupling agents having an aminoalkyl group, such as 3-aminopropyltrimethoxysilane, 3-aminopropyltriethoxysilane, 3-aminopropyltributoxysilane, and 4-aminobutyltriethoxysilane; silane coupling agents having a phenylalkyl group, such as N-phenyl-3-aminopropyltrimethoxysilane; silane coupling agents having a dialkylaminoalkyl group, such as diethylaminomethyltriethoxysilane and (N,N-diethyl-3-aminopropyl)trimethoxysilane; silane coupling agents having a quaternary ammonium group, such as N-trimethoxysilylpropyl-N,N,N-trimethylammonium chloride; silane coupling agents having a carboxyl group, such as carboxyethylsilanetriol, triethoxysilylpropylmaleamic acid, and N-(trimethoxysilylpropyl)ethylenediamine triacetic acid; and silane coupling agents having a sulfoxyl group, such as 3-(trihydroxysilyl)-1-propanesulfonic acid.

The treatment of porous silica particles with a silane coupling agent is not particularly limited, and a known treatment method can be employed. For example, a particular silane coupling agent is added to powdery porous silica particles, the resulting mixture is stirred under heating according to need, and then filtering, drying, etc. are conducted. Thus, a support for protein immobilization can be obtained.

In the treatment with a silane coupling agent having an anion-exchange group such as an amino group or a quaternary ammonium group, an appropriate base such as aqueous ammonia may be added to a reaction mixture in the treatment reaction. In the treatment with a silane coupling agent having a cation-exchange group such as a carboxyl group, an appropriate acid such as acetic acid may be added to a reaction mixture in the treatment reaction.

The porous silica particles used as a raw material may be dispersed in advance in an appropriate polar solvent such as ethanol, and may be used for the treatment. In many cases, the treatment of the porous silica particles can be conducted in air. Alternatively, the treatment may be conducted in an inert gas atmosphere according to need. Conditions such as the heating temperature and the treatment time can be appropriately determined in accordance with properties of the porous silica particles and an organic acid.

As for an embodiment of the treatment, a silane coupling agent and, optionally, an acid or a base may be added to an aqueous dispersion liquid of porous silica particles. Alternatively, porous silica particles or a dispersion liquid thereof may be added to a silane coupling agent-containing aqueous solution to which an acid or a base is optionally added.

Examples of the organic acid used in the method for producing a support for protein immobilization according to the present invention include dicarboxylic acids such as adipic acid, succinic acid, glutaric acid, pimelic acid, suberic acid, azelaic acid, and sebacic acid.

The treatment of the porous silica particles with the organic acid is not particularly limited, and a known treatment method can be employed. For example, a particular organic acid is added to (powdery) porous silica particles having an amino group on the surfaces thereof, the porous silica particles being dispersed in an appropriate aqueous solvent, the resulting mixture is stirred under heating according to need, and then filtering, drying, etc. are conducted. Thus, a support for protein immobilization can be obtained. The porous silica particles used as a raw material may be dispersed in advance in an appropriate polar solvent such as ethanol, and may be used for the treatment. The porous silica particles having an amino group on the surfaces thereof are obtained by treating porous silica particles having a silanol group on the surfaces thereof with a silane coupling agent having an amino group. In many cases, this treatment of the porous silica particles can be conducted in air. Alternatively, the treatment may be conducted in an inert gas atmosphere according to need. Conditions such as the heating temperature and the treatment time can be appropriately determined in accordance with properties of the porous silica particles and the organic acid.

2. Immobilized protein

2-1. Immobilized protein

In the present invention, a protein is immobilized on the above support for protein immobilization, and the support is used in the form of an immobilized protein. That is, an immobilized protein according to the present invention is obtained by immobilizing a protein on the above-described support for protein immobilization.
In a preferred embodiment of the present invention, this immobilized protein is an immobilized enzyme obtained by immobilizing an enzyme as the protein. However, this does not mean that the protein constituting the immobilized enzyme is not limited to an enzyme. The immobilized protein may be an immobilized protein obtained by immobilizing another type of protein such as an antibody on the support for protein immobilization.

Protein

The type of protein used in the immobilized protein according to the present invention is not particularly limited. However, from the standpoint of applications to the industrial use, for example, the use in a catalytic reaction, a preferred protein is an enzyme.
Examples of the enzyme applied to the immobilized protein according to the present invention include, but are not limited to, oxidoreductases that catalyze a redox reaction, transferases that transfer a functional group or an atomic group between molecules, hydrolases that catalyze a hydrolysis reaction, lyases that catalyze an elimination reaction or an addition reaction, isomerases that catalyze a structural conversion between isomers, and ligases that bind two molecules by utilizing hydrolysis energy of adenosine triphosphate.
Examples of the oxidoreductases include various oxidases and reductases such as an alcohol dehydrogenase involved in a reaction in which an alcohol is converted to an aldehyde, an aldehyde dehydrogenase and an aldehyde oxidase that are involved in a reaction in which an aldehyde is converted to a carboxylic acid, a carbon monoxide dehydrogenase involved in a reaction in which carbon monoxide is converted to carbon dioxide, an L-amino acid oxidase involved in mutual transformation between an L-amino acid and a 2-oxoacid, and an aldose reductase involved in a reaction in which an aldose is converted to a sugar alcohol.
Examples of the transferases include a methyltransferase involved in transfer of a methyl group, a carboxyltransferase involved in transfer of a carboxyl group, a transaldolase involved in transfer of an aldehyde group and a keto group, an acyltransferase involved in transfer of an acyl group, a glycosyltransferase involved in transfer of a glycosyl group, an aminotransferase involved in transfer of an amino group, a phosphotransferase involved in transfer of a phosphate group, and a sulfotransferase involved in transfer of a sulfur group.
Examples of the hydrolases include an esterase involved in hydrolysis of an ester, a protease involved in hydrolysis of a peptide bond, and a glycosidase involved in hydrolysis of a sugar. A typical example of the esterase is lipase. Typical examples of the glycosidase include amylase, lactase, maltase, saccharase, and lysozyme.
Examples of the lyases include a carboxylase and a decarboxylase that are involved in addition and elimination of a carboxyl group, an aldehyde-lyase involved in aldol condensation and a reverse reaction thereof, and a dehydratase. An example of the aldehyde-lyase is deoxyriboaldolase.
Examples of the isomerases include an epimerase and a racemase that are involved in isomerization of an asymmetric center in a substrate. A typical example of the racemase is an amino-acid racemase involved in mutual transformation between an L-amino acid and a D-amino acid.
Examples of the ligases include an asparagine synthetase involved in a reaction that synthesizes L-asparagine from L-aspartic acid and ammonia.
The protein immobilized on the support for protein immobilization according to the present invention may be another type of protein such as an antibody instead of the above enzyme. Alternatively, the protein may be a multienzyme including a plurality of polypeptide chains. Examples of the multienzyme include, but are not limited to, proteasomes and cellulosomes.
A known protein or enzyme can be immobilized on the support for protein immobilization according to the present invention. However, as described above, the support for protein immobilization has a pore size of a peak value in the pore size distribution thereof in the range of 2 to 200 nm, and preferably in the range of 2 to 50 nm. Therefore, more preferably, a protein or an enzyme to be immobilized also has a size within this range. It is recommended that the size of the protein or enzyme be still more preferably in the range of 3 to 90 nm, and particularly preferably in the range of 3 to 45 nm.
In the case where the support for protein immobilization or the support for enzyme immobilization according to the present invention has a silanol group or a cation-exchange group on the surface thereof, the support for protein immobilization or the support for enzyme immobilization is on the acidic side. Accordingly, the support is particularly suitable for immobilizing a protein or an enzyme in an acidic pH environment relative to the isoelectric point (pH at which the net charge becomes zero) of the protein in a charge curve that is specific to the protein and represented by a correlation chart between the net charge of the protein and the pH.

On the other hand, in the case where the support for protein immobilization or the support for enzyme immobilization according to the present invention has an anion-exchange group on the surface thereof, the support for protein immobilization or the support for enzyme immobilization is on the basic side. Accordingly, the support is particularly suitable for immobilizing a protein or an enzyme in a basic pH environment relative to the isoelectric point of the protein.

In the case where the support for protein immobilization or the support for enzyme immobilization according to the present invention has a hydrophobic organic group on the surface thereof, the support is particularly suitable for immobilizing a highly hydrophobic protein or enzyme.

2-2. Method for producing immobilized protein

The method for producing an immobilized protein according to the present invention is not particularly limited as long as the objects of the present invention can be achieved. The immobilized protein according to the present invention can be usually produced by immobilizing a desired protein on the above-described support for protein immobilization.

In a preferable method, the immobilized protein according to the present invention can be produced by allowing a protein to adsorb onto the support for protein immobilization. According to this method, since the protein can be immobilized by the simplest procedure, industrial mass production can be advantageously realized. Furthermore, since a wide range of types of functional groups can be used as the functional group present on the surface of the support for protein immobilization, this method is advantageous in that immobilization of various types of proteins can be realized. Typically, the immobilized protein can be produced by allowing a protein to adsorb onto the support for protein immobilization in an appropriate buffer solution. The reaction conditions such as the pH and the temperature in this step can be appropriately adjusted in accordance with properties of the protein and the porous silica particles constituting the support for protein immobilization. For example, from the standpoint of suppressing denaturation of the protein to the minimum, the temperature may be 0°C to 10°C, for example, about 4°C. From the standpoint of rapidly conducting the reaction within a range in which denaturation of the protein does not occur, the temperature may be 20°C to 40°C, for example, about 30°C to 37°C. However, in many cases, the adsorption of the protein to the support for protein immobilization is conducted at about room temperature, and typically in the range of 4°C to 25°C.

Herein, the term "adsorption" refers to a concept that immobilization performed by an interaction related to a hydrogen bond, an electrostatic interaction, or an affinity, or by another interaction related to a non-covalent bond can also be included.

Here, it is believed that, in the immobilized protein of the present invention, immobilization is presumably achieved because a hydrogen bond is interposed between a silanol group on the surface of the support for protein immobilization and a protein. In the case where the support for protein immobilization is composed of a porous silica particle having an anion-exchange group or a cation-exchange group on the surface thereof, an electrostatic interaction can also be advantageously utilized in immobilization of a protein.

Meanwhile, in the case where the support for protein immobilization is composed of a porous silica particle having a functional group which can form a covalent bond with a protein, such as an amino group or a carboxyl group, on the surface thereof, in order to achieve stronger immobilization, the protein may be immobilized on the support for protein immobilization by forming a covalent bond by a usual method as long as the function of the protein is not impaired.

2-3. Use of immobilized protein

The immobilized protein of the present invention is mainly used as an immobilized enzyme. In the case where the immobilized protein of the present invention is used as an immobilized enzyme, for example, the following advantages can be achieved: The reaction activity of the immobilized enzyme to a substrate is equivalent to that of a corresponding free enzyme, i.e., a corresponding free enzyme that is not supported. In addition, a decrease in the activity due to a repeated use is smaller than that of the corresponding free enzyme.

In the case where an enzyme is used as a protein immobilized, the resulting immobilized enzyme is used in the same applications as those of a corresponding free enzyme that is not supported (hereinafter may be referred to as "free enzyme").

For example, the immobilized protein of the present invention can be used, as an immobilized enzyme in which the oxidoreductase, the transferase, the hydrolase, the lyase, the isomerase, or the ligase described above is immobilized, in various applications including the industrial use. For example, the immobilized protein can be used, as an immobilized enzyme in which an isomerase such as a racemase is immobilized, in an isomerization reaction of an amino acid or the like. Alternatively, the immobilized protein can be used, as an immobilized enzyme in which an aldehyde-lyase such as deoxyriboaldolase is immobilized, in an aldol reaction or the like.

[EXAMPLES]

[Methods for measuring physical properties]

1. Method for measuring average particle diameter

(A) Measurement of average particle diameter by image analysis method

The average particle diameter of spherical silica fine particles was measured with a scanning electron microscope and an image analysis apparatus as described in the section of "5. Measurement of particle size distribution" below.

(B) Method for measuring average particle diameter by centrifugal sedimentation method

The average particle diameter of porous silica particles was measured as follows. First, a dispersion liquid of porous silica particles (water or a 40% by mass glycerin solvent, solid concentration: 0.1 to 5% by mass) was dispersed in an ultrasonic generator (produced by Iuchi Seieido Co., Ltd., Model US-2) for five minutes. Furthermore, water or glycerin was added to the dispersion liquid to appropriately adjust the concentration. The resulting dispersion liquid was put in a glass cell (having a size of 10 mm in length, 10 mm in width, and 45 cm in height), and the average particle diameter was measured using a centrifugal sedimentation-type particle size distribution analyzer (produced by HORIBA, Ltd.: CAPA-700).

2. Method for measuring specific gravity

The specific gravity of porous silica particles was measured by the following method.
First, 10 g of a sample was put in a crucible and was dried at 110°C for two hours. Next, this dry sample was cooled in a desiccator, and 3 to 4 g of the sample was then put in a 25-mL pycnometer. Distilled water was added thereto to suspend the sample, and vacuum degassing was performed at 60 mmHg for one hour. The temperature of the resulting suspension was then adjusted in a thermostatic chamber at 25°C. The volume was adjusted by adding distilled water up to a marked line of the pycnometer. The volume (mL) of the sample was calculated from the difference between the volume (25 mL) of the pycnometer and the volume (mL) of distilled water. The specific gravity was determined from the weight (g) of the sample added and the volume (mL) calculated as described above.

3. Method for measuring porosity

The porosity of porous silica particles was calculated from the formula below using the specific gravity determined in 2. above and the specific gravity of silica (i.e., the apparent specific gravity of silica in which gaps present inside thereof are not considered).
100 - [Specific gravity of porous silica particles determined in 2. above]/[Specific gravity of silica] x 100 = Porosity (%)

4. Method for measuring sphericity

In a photographic projection image obtained by taking a photograph of an oxide sol sample at a magnification of 250,000 with a transmission electron microscope (produced by Hitachi Ltd., H-800), a ratio (DS/DL) of a maximum diameter (DL) to a short diameter (DS) orthogonal to the maximum diameter was measured for 50 arbitrary particles, and the average of the values of the ratio was defined as a sphericity.

5. Measurement of particle size distribution

An image of particles was taken with a scanning electron microscope (produced by JEOL, Ltd., Model JSM-5300) (magnification: 250,000). The average particle diameter of 250 particles in this image was measured using an image analysis apparatus (produced by Asahi Kasei Corporation, IP-1000), and the coefficient of variation (CV value) related to the particle diameter distribution was calculated. Specifically, the particle diameter of each of the 250 particles was measured, and the average particle diameter and the standard deviation of the particle diameter were determined from the values of the particle diameter. The coefficient of variation (CV value) was calculated from the formula below.

$$\text{Coefficient of variation (CV value)} = \text{(Standard deviation } (\sigma) \text{ of particle diameter/Average particle diameter } (Dn)) \times 100 \ (\%).$$

Coefficient of variation (CV value) = (Standard deviation ($\sigma$) of particle diameter/Average particle diameter (Dn)) $\times$ 100 (%).

6. Method for measuring pore volume and pore size

A gas adsorption method and a mercury penetration method were used as methods for measuring the pore volume and the pore size of porous silica particles. In Examples, Reference Example, and Comparative Examples described below, the measurement was conducted by employing a gas adsorption method unless otherwise stated.

The measurement of the pore volume and the pore size by the gas adsorption method was conducted in accordance with the following procedure.

The pore volume of porous silica particles was measured as follows: Ten grams of a sample was put in a crucible, dried at 300°C for one hour, and then cooled to room temperature in a desiccator. Next, 0.15 g of the sample was put in a glass cell. Nitrogen gas was allowed to adsorb onto the sample while performing vacuum degassing using a Belsorp mini II (produced by BEL Japan, Inc.) and was then allowed to desorb. From the obtained adsorption isotherm, the pore volume was determined at a point having a relative pressure of 0.990, and the pore size (peak value) was calculated by a BJH method.

Regarding the measurement of the pore volume and the pore size by the mercury penetration method, 10 g of a sample was put in a crucible, dried at 300°C for one hour, then cooled to room temperature in a desiccator, and the measurement was conducted using PM-33 (produced by Quantachrome Instruments). In this measurement, mercury was penetrated at 3.5 kPa to 231 MPa (0.5 to 33,000 psi), and the pore distribution was determined from the relationship among the pressure, the pore size, and the amount of penetration. According this method, mercury is penetrated into pores having a size of about 5.4 nm to about 5.4 $\mu$m, and the measurement is conducted. Accordingly, both pores present inside the porous silica particles and gaps between the porous silica particles are measured. Regarding the volume of only the pores present inside the porous silica particles, the pore volume and the pore size were calculated on the basis of the measurement results of pores having a pore size of 200 nm or less.

[Raw material porous silica particles]

In Examples of the present invention, SILICA MICRO BEAD P-7H, P-12H, P-4H, and P-20H produced by JGC Catalysts and Chemicals Ltd. were respectively used as porous silica particles (hereinafter referred to as "raw material porous silica particles") P1, P2, P3, and P4 serving as the raw materials of a support for enzyme immobilization.

The raw material porous silica particles P1, P2, P3, and P4 were produced by a spray-drying method.

These raw material porous silica particles were produced by the following procedure.

A spherical silica fine particle dispersion liquid (silica concentration: 30% by mass) in which spherical silica fine particles are dispersed in water was centrifuged to prepare a spherical silica fine particle dispersion liquid from which coarse particles were removed. The solid concentration of this spherical silica fine particle dispersion liquid was adjusted to 15% by mass to prepare a spray liquid. Spray drying was then conducted under the conditions of an inlet temperature of 220°C and an outlet temperature of 50°C. Thus, raw material porous silica particles were obtained.

In contrast, raw material porous silica particles G1 were produced by crushing silica, and then making the resulting particles fine by a spray-drying method.

Table 1 describes characteristics of the raw material porous silica particles (P1 to P4 and G1).

[EXAMPLE 1]

[Preparation of supports for enzyme immobilization (supports modified with amino group)]

1. Preparation of support P-3N for enzyme immobilization

Forty grams of the powdery porous silica particles P3 were dispersed in 155 g of ethanol in air. The resulting dispersion liquid was stirred for five minutes, 13 g of pure water was then added thereto, and stirring was further conducted for 30 minutes.

Next, 6 g of a silane coupling agent (produced by Shin-Etsu Chemical Co., Ltd., "KBM-903" (3-aminopropyltrimethoxysilane)) was added to the dispersion liquid in air and the resulting reaction mixture was stirred for 30 minutes. Subsequently, 0.36 g of a 29% aqueous ammonia solution was added thereto and stirring was conducted for 30 minutes. Subsequently, the temperature was increased to 50°C, and stirring was continued at 50°C for 20 hours. The reaction mixture was then cooled to room temperature, a solid portion was collected by filtering, and the solid portion was dried at 150°C for two hours. Thus, about 35 g of a support P-3N for enzyme immobilization was obtained.

2. Preparation of supports P-1N, P-2N, P-4N, and G-1N for enzyme immobilization

Supports P-1N, P-2N, P-4N, and G-1N for enzyme immobilization were prepared by the same method as the method for preparing the support P-3N for enzyme immobilization except that the porous silica particles P1, P2, P4, and G1 were respectively used as the raw materials instead of the porous silica particles P3.
Table 1 describes characteristics of the prepared supports (P-1N, P-2N, P-3N, P-4N, and G-1N) for enzyme immobilization.

[EXAMPLE 2]

[Preparation of supports for enzyme immobilization (supports modified with carboxyl group)]

1. Preparation of support P-3C for enzyme immobilization

Forty grams of the powdery porous silica particles P-3N were dispersed in 155 g of ethanol in air. The resulting dispersion liquid was stirred for five minutes, 13 g of pure water was then added thereto, and stirring was further conducted for 30 minutes.
Next, 6 g of adipic acid [produced by Kanto Chemical Co., Inc.] was added to the dispersion liquid in air and the resulting reaction mixture was stirred for 30 minutes. Subsequently, the temperature was increased to 50°C, and stirring was continued at 50°C for 20 hours. The reaction mixture was then cooled to room temperature, a solid portion was collected by filtering, and the solid portion was dried at 150°C for two hours. Thus, about 35 g of a support P-3C for enzyme immobilization was obtained.

2. Preparation of supports P-1C, P-2C, P-4C, and G-1C for enzyme immobilization

Supports P-1C, P-2C, P-4C, and G-1C for enzyme immobilization were prepared by the same method as the method for preparing the support P-3C for enzyme immobilization except that the porous silica particles P1, P2, P4, and G1 were respectively used as the raw materials instead of the porous silica particles P3.
Table 1 describes characteristics of the prepared supports (P-1C, P-2C, P-3C, P-4C, and G-1C) for enzyme immobilization.

[EXAMPLE 3]

[Preparation of supports for enzyme immobilization (supports modified with phenylamino group)]

1. Preparation of support P-3Ph for enzyme immobilization

Forty grams of the powdery porous silica particles P3 were dispersed in 155 g of ethanol in air. The resulting dispersion liquid was stirred for five minutes, 13 g of pure water was then added thereto, and stirring was further conducted for 30 minutes.
Next, 6 g of a silane coupling agent (produced by Shin-Etsu Chemical Co., Ltd., "KBM-573" (N-phenyl-3-aminopropyltrimethoxysilane)) was added to the dispersion liquid in air and the resulting reaction mixture was stirred for 30 minutes. Subsequently, 0.36 g of a 29% aqueous ammonia solution was added thereto and stirring was conducted for 30 minutes. Subsequently, the temperature was increased to 50°C, and stirring was continued at 50°C for 20 hours. The reaction mixture was then cooled to room temperature, a solid portion was collected by filtering, and the solid portion was dried at 150°C for two hours. Thus, about 35 g of a support P-3Ph for enzyme immobilization was obtained.

2. Preparation of supports P-1Ph, P-2Ph, P-4Ph, and G-1Ph for enzyme immobilization

Supports P-1Ph, P-2Ph, P-4Ph, and G-1Ph for enzyme immobilization were prepared by the same method as the method for preparing the support P-3Ph for enzyme immobilization except that the porous silica particles P1, P2, P4, and G1 were respectively used as the raw materials instead of the porous silica particles P3.

Table 1 describes characteristics of the prepared supports (P-1Ph, P-2Ph, P-3Ph, P-4Ph, and G-1Ph) for enzyme immobilization.

[REFERENCE EXAMPLE 1]

[Supports for enzyme immobilization (supports having untreated silanol group)]

Regarding supports for enzyme immobilization, the supports having an untreated silanol group, the porous silica particles P1, P2, P3, P4, and G1 were respectively used as supports P-1, P-2, P-3, P-4, and G-1 for enzyme immobilization as they were without conducting a surface modification treatment with a chemical agent such as a silane coupling agent.

[COMPARATIVE EXAMPLE 1]

FSM-type porous silica particles used as comparative examples were prepared by a known method described in a literature (for example, Japanese Unexamined Patent Application Publication No. 2004-83501). Specifically, an appropriate surfactant (swelling agent) was added to an aqueous dispersion liquid of $\delta$-$Na_2Si_2O_5$ (kanemite), and the mixture was allowed to react under heating. The reaction mixture was filtered, and then washed with water. The resulting product was dried in air and then baked to prepare FSM-type porous silica particles. Here, FSM-type porous silica particles having a pore size of 4.0 nm, 4.2 nm, 7.5 nm, 8.0 nm, 8.5 nm, and 9.2 nm were obtained by using 1,3,5-triisopropylbenzene as the swelling agent in an amount of 0 mL, 0 mL, 4.5 mL, 5.5 mL, 6.0 mL, and 11.2 mL, respectively.

Table 1 also describes characteristics of the FSM-type porous silica particles used as the comparative examples. These FSM-type porous silica particles were used as supports for enzyme immobilization as they were without particularly conducting a surface modification treatment with a chemical agent such as a silane coupling agent.

[Table 1]

[Table 1]

(A-1) Raw material porous silica particles used in Examples

| Name | Physical properties at untreated stage | | | | | | | | | | |
|---|---|---|---|---|---|---|---|---|---|---|---|
| | Porous silica particles | | | | | | | Silica fine particles | | | |
| | Pore size | Average particle diameter | Specific surface area | Pore volume | Pore size (D1) of peak value in pore size distribution | Ratio of pore volume in (D1) × 0.75 to (D1) × 1.25 to total maximum volume | Porosity | Average particle diameter | Sphericity | Coefficient of variation of particle diameter | Whether particle diameter distribution is monodispersion phase or not |
| | nm | μm | m²/g | mL/g | nm | % | % | nm | - | % | |
| P3 | 4.0 | 3 | 201 | 0.15 | 4.0 | 90 | 33 | 12 | 0.95 | 3 | Monodispersion phase |
| P1 | 7.0 | 3 | 111 | 0.15 | 7.0 | 90 | 34 | 17 | 0.94 | 3 | Monodispersion phase |
| P2 | 12.0 | 3 | 75 | 0.17 | 12.0 | 94 | 35 | 25 | 0.91 | 3 | Monodispersion phase |
| P4 | 20.0 | 3 | 64 | 0.21 | 20.0 | 91 | 35 | 45 | 0.93 | 3 | Monodispersion phase |
| G1 | 8.0 | 3 | 151 | 0.32 | 8.0 | 85 | 40 | 20 | 0.91 | 5 | Monodispersion phase |

(A-2) Supports for enzyme immobilization used in Examples

| Raw material porous silica particles | Support for enzyme immobilization | | | |
|---|---|---|---|---|
| | Type of surface treatment | | | |
| | No surface treatment (Particle surface has silanol group) | Modified with amino group | Modified with carboxyl group | Modified with phenylamino group |
| P3 | P-3 | P-3N | P-3C | P-3Ph |
| P1 | P-1 | P-1N | P-1C | P-1Ph |
| P2 | P-2 | P-2N | P-2C | P-2Ph |
| P4 | P-4 | P-4N | P-4C | P-4Ph |
| G1 | G-1 | G-1N | G-1C | G-1Ph |

(B) FSM-type porous silica particles used in Comparative Examples

| Name | Physical properties at untreated stage | | | | | | |
|---|---|---|---|---|---|---|---|
| | Porous silica particles | | | | | | |
| | Pore size | Average particle diameter | Specific surface area | Pore volume | Pore size (D1) of peak value in pore size distribution | Ratio of pore volume in (D1) × 0.75 to (D1) × 1.25 to total maximum volume | Remarks |
| | nm | $\mu$m | m$^2$/g | mL/g | nm | % | |
| FSM-4.0 | 4.0 | Irregular shape (5 to 15 $\mu$m) | 1,080 | 1.3 | 4 | 91.4 | Do not have an interparticle gap structure inside. |
| FSM-4.2 | 4.2 | Irregular shape (5 to 15 $\mu$m) | 1,120 | 1.55 | 4.16 | 86.1 | |
| FSM-7.5 | 7.5 | Irregular shape (5 to 15 $\mu$m) | 1,080 | 2.3 | 7.5 | 61.8 | |
| FSM-8.0 | 8.0 | Irregular shape (5 to 15 $\mu$m) | 1,120 | 2.19 | 8 | 65.6 | |
| FSM-8.5 | 8.5 | Irregular shape (5 to 15 $\mu$m) | 1,100 | 2.57 | 8.5 | 57.4 | |
| FSM-9.2 | 9.2 | Irregular shape (5 to 15 $\mu$m) | 1,100 | 2.19 | 9.2 | 57.5 | |

[EXAMPLE 4]

1. Preparation of immobilized enzyme (racemase-adsorbed enzyme) and measurement of the amount of enzyme adsorbed

A buffer solution (a mixture of 20 mM potassium phosphate and 50 mM potassium chloride, pH 7.5) and a racemase solution in which a racemase was dissolved in the buffer solution (racemase concentration: 2 mg/mL) were prepared.

Next, 1 mL of the buffer solution, 1 mL of the racemase solution, and 10 mg of a support for enzyme immobilization were mixed to prepare a mixed solution. This mixed solution was stored in an incubator at a temperature of 4°C for 24 hours to prepare an immobilized enzyme in which the racemase was adsorbed on the support for enzyme immobilization.

Subsequently, the mixed solution was centrifuged with a centrifugal separator [produced by Eppendorf Co., Ltd., product number: Centrifuge 5417R] (at 21,000G, for one minute) to precipitate the immobilized enzyme. The amount of racemase in the supernatant was measured by a bicinchoninic acid (BCA) method, and the measured value was determined as the amount of unadsorbed racemase. The value calculated by subtracting the amount of unadsorbed racemase from the amount (2 mg) of racemase initially charged was determined as the amount of racemase adsorbed on the support for enzyme immobilization.

This experiment was conducted using each of the supports for enzyme immobilization prepared in Examples 1 to 3.

2. Method for assaying protein by bicinchoninic acid (BCA) method

The assay of the racemase was conducted using a commercially available assay reagent kit (produced by Thermo Fisher Scientific K.K., "BCA Protein Assay Reagent").

3. Test of specific activity of immobilized enzyme (racemase)

[Sample]

An immobilized enzyme (in an amount corresponding to the case where 2 $\mu$g of a racemase was adsorbed on a support for enzyme immobilization) and 2.5 mL of a substrate solution (10 mM L-alanine and 10 mM potassium phosphate, pH 7.5) were mixed in a test tube, and the resulting mixture was allowed to react under the condition of a temperature of 30°C, thus obtaining a suspension in which a very small amount of silica particles were floated in a transparent aqueous solution. This suspension was used as a sample.

[Measurement of specific activity based on rate of change in circular dichroism of L-Ala with time]

The sample of the suspension was put in a quartz cell having a bottom area of 1 square centimeter, and the cell was set in a circular dichroism dispersion meter (produced by JASCO Corporation, "J-820"). The suspension in the cell was stirred with a magnetic stirrer, and the temperature of the cell was maintained at 30°C with a Peltier temperature controller.

Next, the ellipticity at a wavelength of 204 nm of circularly polarized light of L-alanine in the circular dichroism dispersion meter was measured for five minutes at intervals of 1 second. The concentration of L-alanine in the suspension was measured on the assumption that the molar ellipticity of L-alanine was 23 mdeg/mM. The activity was calculated from the amount of alanine converted per unit time. The specific activity was determined by the following formula.

Specific activity = [Activity of enzyme immobilized on mesoporous silica]/[Activity of enzyme before immobilization]

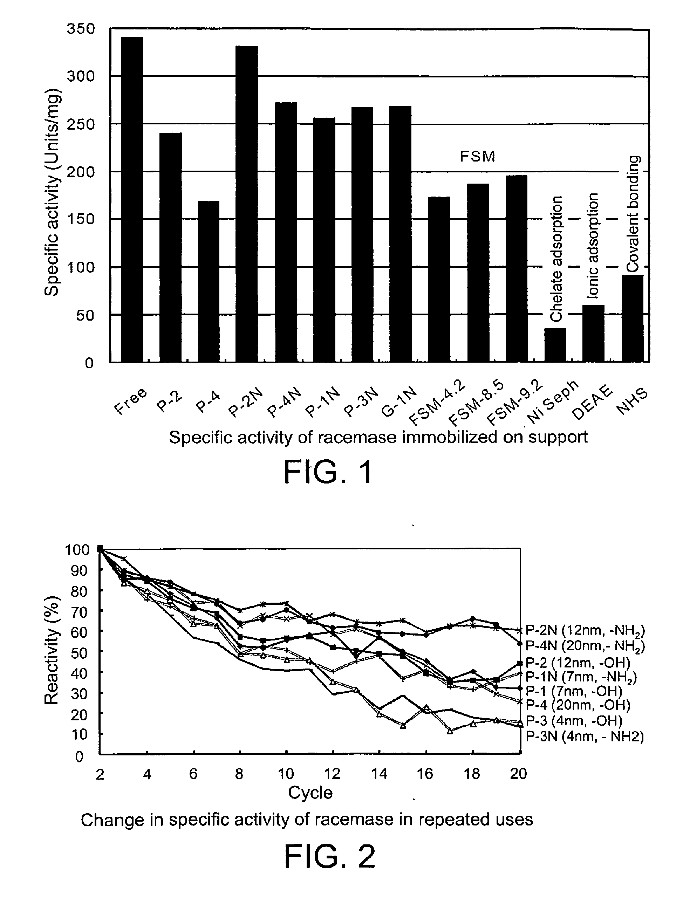

The specific activities of the immobilized enzymes (P-2, P-4, P-2N, P-4N, P-1N, P-3N, and G-1N) are illustrated in FIG. 1.

FIG. 1 also illustrates the specific activities of immobilized enzymes described below as comparative examples. The measurement of the amount of racemase adsorbed on the support for enzyme immobilization, and other measurements were conducted under the same conditions as those described above.

1) An immobilized enzyme ("DEAE") prepared by immobilizing the racemase on a support ("DEAE Sepharose" produced by GE Healthcare Japan Corporation) by an ion-exchange method.

2) An immobilized enzyme ("Ni Seph.") prepared by immobilizing the racemase on a support ("Ni Sepharose 6 Fast Flow" produced by GE Healthcare Japan Corporation) by a metal chelating method.

3) An immobilized enzyme ("NHS") prepared by immobilizing the racemase on a support ("NHS-activated Sepharose 4 Fast Flow" produced by GE Healthcare Japan Corporation) by a covalent bond method.

4) An immobilized enzyme ("FSM-4.2") prepared by immobilizing the racemase on an FSM having a pore size of 4.2 nm.

5) An immobilized enzyme ("FSM-8.5") prepared by immobilizing the racemase on an FSM having a pore size of 8.5 nm.

6) An immobilized enzyme ("FSM-9.2") prepared by immobilizing the racemase on an FSM having a pore size of 9.2 nm.

Referring to FIG. 1, it is found that each of the immobilized enzymes according to the present invention (P-2, P-4, P-2N, P-4N, P-1N, P-3N, and G-1N exhibits specific activity equal to or higher than that of the immobilized enzymes of the comparative examples.

4. Test of specific activity when immobilized enzyme (racemase) was repeatedly used

(1) An immobilized enzyme (prepared by allowing 2 $\mu$g of a racemase to adsorb onto 3 g of a support for enzyme immobilization) and 1 mL of a substrate solution (5 mM L-Ala, pH 8.5) were mixed in a test tube, and a suspension reaction of the resulting mixture was conducted under the condition of a temperature of 30°C in a Deep Well Maximizer (produced by TAITEC Co., Ltd.) at 1,800 rpm for two minutes, thus obtaining a suspension.

Subsequently, this suspension was centrifuged with a centrifugal separator [produced by Eppendorf Co., Ltd., product number: Centrifuge 5417R] (at 21,000G, for one minute) to precipitate the immobilized enzyme. The supernatant was removed, and centrifugal separation was further conducted under the same conditions. The supernatant was completely removed to collect the immobilized enzyme.

The collected immobilized enzyme and 1 mL of the same substrate solution as that used in the above suspension reaction were mixed in a test tube, and a suspension reaction of the resulting mixture was conducted under the condition of a temperature of 30°C in a Deep Well Maximizer (produced by TAITEC Co., Ltd.) at 1,800 rpm for two minutes, thus obtaining a suspension.

Subsequently, this suspension was centrifuged with a centrifugal separator (at 21,000G, for one minute) to precipitate the immobilized enzyme. Next, the supernatant was separated, and the amount of D-Ala in the supernatant was measured to determine the activity. The above operations were repeated 20 times. The activity of each of the immobilized enzymes was measured, and the specific activity was calculated. The results are illustrated in FIG. 2.

According to the results, it was found that the immobilized enzymes according to the present invention (P-2N, P-4N, P-2, P-1N, P-1, P-4, P-3, and P-3N) could maintain good specific activity even when they were repeatedly used.

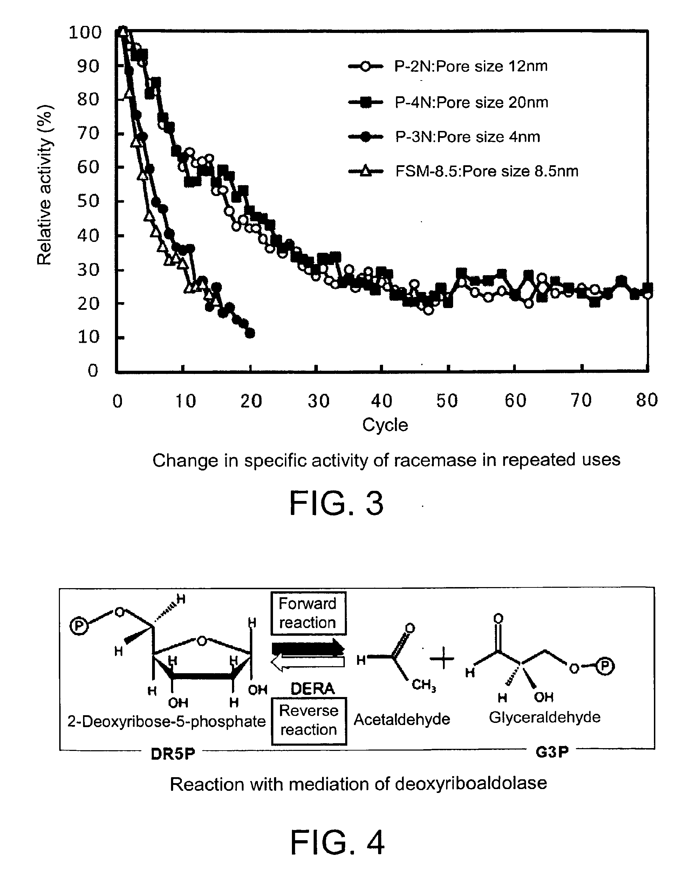

(2) FIG. 3 illustrates the results obtained when the repetitive activity of the immobilized enzymes was examined until 80 cycles had passed. It was confirmed that the immobilized enzymes according to the present invention exhibited a high activity-maintaining property when the enzymes were repeatedly used, as compared with FSM 8.5, which was a comparative example. The reason for this is believed to be as follows. FSM 8.5 is mechanically fractured during centrifugal separation, whereas such a fracture does not occur in the immobilized enzymes according to the present invention. Thus, stable activity could be finally obtained in the immobilized enzymes according to the present invention.

[EXAMPLE 5] (Test of performance of KpDERA-adsorbed-type immobilized enzyme)

Deoxyriboaldolase (DERA) was immobilized on the supports prepared as in Example 4. DERA is an enzyme that mediates the reactions illustrated in FIG. 4.

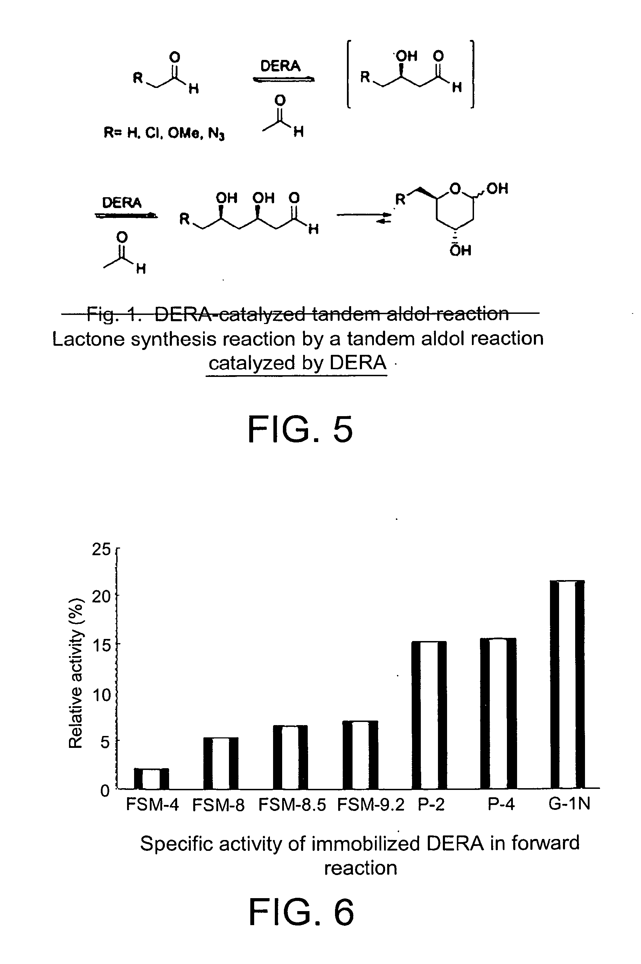

Herein, the reaction in which 2-deoxyribose-5-phosphate (DR5P) is converted to acetaldehyde and glyceraldehyde triphosphate (G3P) is referred to as a forward reaction, and the reaction in which G3P and acetaldehyde are converted to DR5P is referred to as a reverse reaction. DERA is also expected as an enzyme that synthesizes a lactone, which is an intermediate of medicine, by a tandem aldol reaction illustrated in FIG. 5. However, this reaction has not been practically used because of deactivation by an aldehyde.

1. Preparation of immobilized enzyme

A buffer solution (50 mM triethanolamine, pH 7.5) and a KpDERA solution in which deoxyriboaldolase derived from Klebsiella pneumoniae (Kp) (hereinafter referred to as "KpDERA") was dissolved in the buffer solution (KpDERA concentration: 2 mg/mL) were prepared.

Next, 1 mL of the buffer solution, 1 mL of the KpDERA solution, and 20 mg of a support P2 for enzyme immobilization were mixed. The resulting mixed solution was stored at a temperature of 4°C for 24 hours to prepare an immobilized enzyme (KpDERA/support for enzyme immobilization) in the form of a suspension. The immobilized enzyme was similarly prepared using P4 or G1N as a support for enzyme immobilization.

As a comparative example, an immobilized enzyme (KpDERA/FSM) was prepared in the form of a suspension as in the above immobilized enzyme except that 20 mg of the support for enzyme immobilization was changed to 10 mg of FSM-4.0. In addition, the immobilized enzyme was similarly prepared using FSM 8.0, FSM 8.5, or FSM 9.2.

2. Measurement of the amount of enzyme adsorbed in immobilized enzyme

Each of the immobilized enzymes (suspensions) obtained in 1. above was centrifuged with a centrifugal separator [Manufacturer: Eppendorf Co., Ltd., product number: Centrifuge 5417R] (at 21,000G, for one minute) to precipitate the immobilized enzyme. The amount of KpDERA in the supernatant was measured by a [bicinchoninic acid (BCA) method], and the measured value was determined as the amount of unadsorbed KpDERA. The value calculated by subtracting the amount of unadsorbed KpDERA from the amount (2 mg) of KpDERA initially charged was determined as the amount of KpDERA adsorbed on the support for enzyme immobilization.

3. Reaction between immobilized enzyme and substrate [DRSP]

An immobilized enzyme (prepared by allowing 5 $\mu$g of KpDERA to adsorb onto 37.5 $\mu$g of a support for enzyme immobilization) and 2.0 mL of a substrate solution (1 mM 2-deoxyribose-5-phosphate [DRSP]) were allowed to react at a temperature of 30°C, thus obtaining a reaction liquid.

This reaction liquid contains triosephosphate isomerase, glyceraldehyde-3-phosphate dehydrogenase, and 0.2 mM NADH. By measuring a coupling reaction from a change in the absorbance (wavelength: 340 nm) of NADH, the amount of change in DR5P was indirectly calculated (forward reaction). Activity that decomposes 1 micromole of the substrate for one minute was defined as 1 unit.

A UV-Vis spectrophotometer (manufacturer: Shimadzu Corporation, model No. UV-2450) was used in the measurement of the absorbance.

<u>4. Reaction between immobilized enzyme and substrate [DG3P]</u>

An immobilized enzyme (prepared by allowing 1.05 μg of KpDERA to adsorb onto 18.4 μg of each support for enzyme immobilization) was allowed to react with a 350 μL a substrate solution [100 mM triethanolamine (pH 7.5), 100 mM DL-glycerol 3-phosphate, and 300 mM acetaldehyde] at 25°C for 20 minutes to obtain a reaction liquid.

Next, 40 μL of the reaction liquid was transferred to another tube, and centrifuged (at 21,000G, for two minutes) to precipitate the immobilized enzyme.

Next, 20 μL of the supernatant was transferred to a new tube, and 8 μL of 60% perchloric acid was added thereto. The resulting mixture was treated on ice for ten minutes.

Subsequently, neutralization was conducted by adding 13.4 μL of 1 M sodium hydroxide and 179 μL of 1 M triethanolamine (pH 7.5), and the amount of DR5P produced was then measured by a cysteine-sulfuric acid method (reverse reaction).

As illustrated in FIG. 6, DERA immobilized on P-4, P-2, and G-1N exhibited specific activity in the forward reaction higher than that in the cases of DERA immobilized on FSMs. On the other hand, in the reverse reaction, as described in Table 2, P-4 maintained relatively high specific activity and exhibited specific activity slightly higher than that in the case of FSM-9.2.

[Table 2]

| Table 2 Specific activity of immobilized DERA | | |
|---|---|---|
| Support | Forward reaction | Reverse reaction |
| Free | 100 | 100 |
| FSM-9.2 | 7.1 | 70.5 |
| P-4 | 15.6 | 74.0 |

<u>5. Confirmation of tolerance to acetaldehyde of immobilized DERA</u>

In order to examine the improvement in tolerance to acetaldehyde of immobilized DERA, first, the effect of acetaldehyde on the forward reaction of the free enzyme ("Free"), which was not supported on a support, was evaluated.

First, 0.2 units of KpDERA was incubated in 300 mM acetaldehyde at 4°C for a certain period. The aldehyde was removed through Spin Column (Thermo Fisher Scientific K.K.), and the residual activity was then determined by measuring the forward reaction.

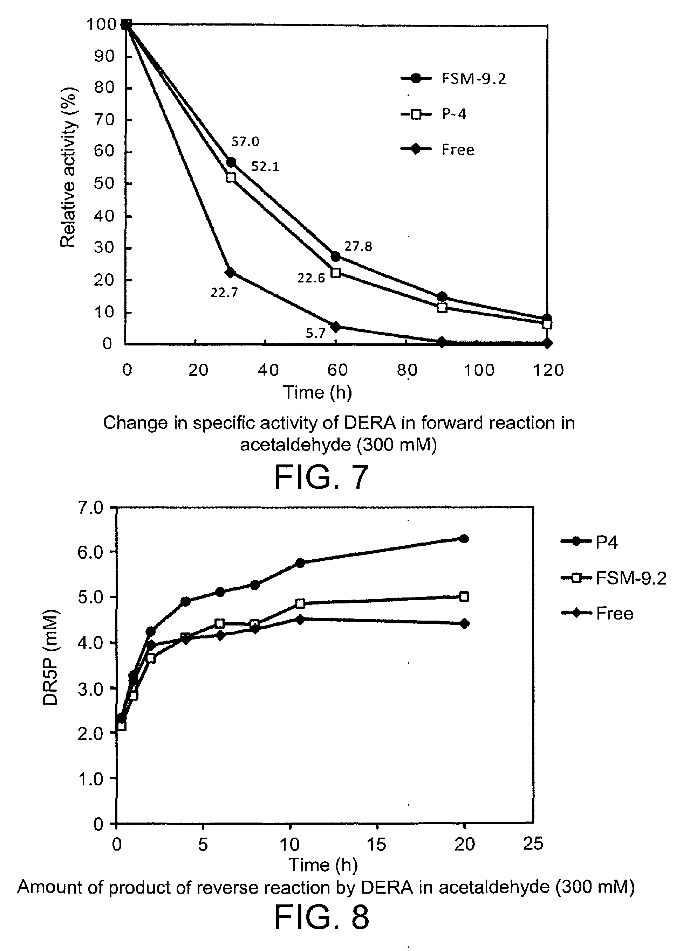

As illustrated in FIG. 7, in an acetaldehyde concentration of 300 mM or more, KpDERA was substantially deactivated after one hour.

<u>6. Confirmation of effect of acetaldehyde on forward reaction of immobilized enzyme</u>

The effect of acetaldehyde on the forward reaction of an immobilized enzyme was evaluated.

An immobilized enzyme according to the present invention on which 0.2 units of KpDERA was adsorbed was incubated in 300 mM acetaldehyde at 4°C for a certain period. The support was washed with 50 mM TEA (pH 7.5), and the residual activity was then determined by measuring the forward reaction.

As illustrated in FIG. 7, both DERA immobilized on FSM-9.2 and DERA immobilized on P-4 exhibited improved acetaldehyde tolerance, as compared with free DERA, which was not supported on a support.

<u>7. Confirmation of effect of acetaldehyde on reverse reaction of immobilized enzyme</u>

The effect of acetaldehyde on the reverse reaction of an immobilized enzyme was evaluated.

An immobilized enzyme according to the present invention on which 0.2 units of KpDERA was adsorbed was allowed to react with 350 μL of a substrate solution (100 mM triethanolamine [pH 7.5], 100 mM DL-glycerol 3-phosphate, and 300 mM acetaldehyde) at 25°C for a certain period.

Next, 25 μL of the reaction liquid was transferred to a new tube and centrifuged (at 21,000G, for two minutes) to precipitate the support. Next, 20 μL of the supernatant was transferred to a new tube, and 8 μL of 60% perchloric acid was added thereto. The resulting mixture was treated on ice for ten minutes. After neutralization was conducted by adding 13.4 μL of 1 M sodium hydroxide and 179 μL of 1 M triethanolamine pH 7.5, the amount of DR5P produced was measured by a cysteine-sulfuric acid method.

As illustrated in FIG. 8, in the reverse reaction, the improvement in the tolerance in P-4 was significantly observed.

8. Application to lactone synthesis reaction

Lactones are important substances as intermediates of medicine. In order to simplify a synthetic route, the synthesis of a lactone by a tandem aldol synthesis reaction catalyzed by DERA and illustrated in FIG. 2.2 has been expected. However, this reaction has not been practically used because of deactivation of DERA by an aldehyde. Accordingly, to examine this reaction, the amounts of lactone precursor (2,4,6-trideoxy-D-erythro-hexapyranoside) produced by a tandem aldol reaction from acetaldehyde were compared.

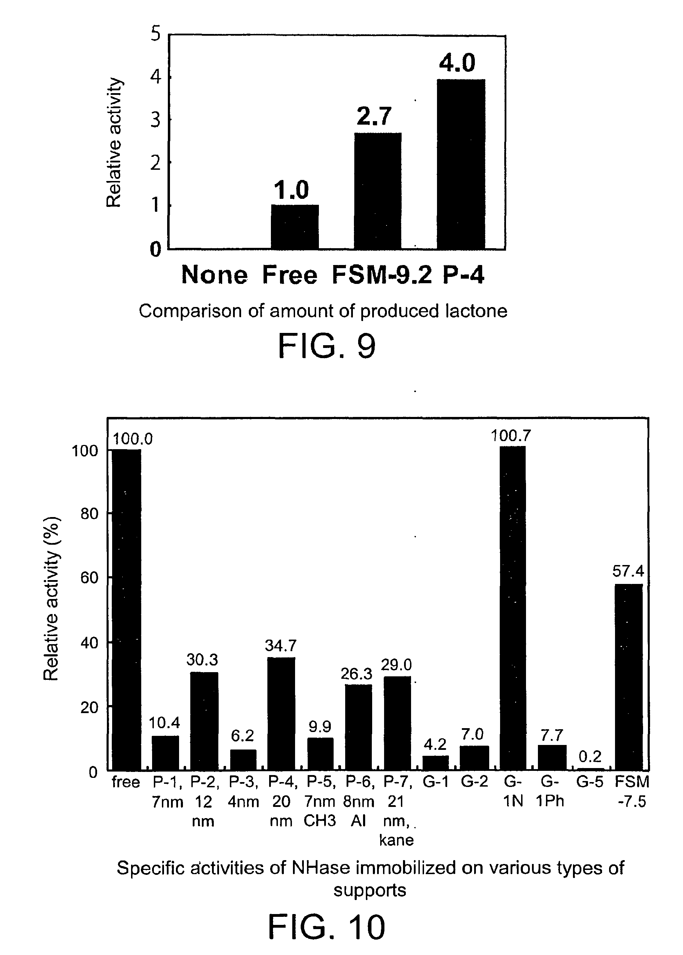

Mesoporous silica of an FSM or immobilized enzyme according to the present invention on which 50 $\mu$g of KpDERA was allowed to adsorb was added to 1 mL of a 500 mM acetaldehyde solution, and the mixture was allowed to react at 25°C for 24 hours. The reaction mixture was centrifuged (at 21,000G, for one minute), and 4 $\mu$L of the supernatant was spotted on a thin-layer chromatography plate (Silica Gel 60, Merck), developed with 1-butanol:acetic acid:water = 4:1:1 (vol/vol/vol), and then subjected to color development using a p-anisaldehyde ethanol solution. The results are illustrated in FIG. 9.

In the system where DERA immobilized on P-4 was used, 2,4,6-trideoxy-D-erythro-hexapyranoside was produced in an amount four times that in the system where free DERA, which was not immobilized on a support, was used. According to these results, it was confirmed that the tolerance to aldehyde, which is a substrate, was improved by immobilizing DERA on P-4.

[EXAMPLE 6] (Test of performance of nitrile hydratase-adsorbed-type immobilized enzyme)

1. Preparation of immobilized enzyme

A buffer solution (a mixture of 20 mM potassium phosphate and 50 mM potassium chloride, pH 7.5) and a nitrile hydratase solution in which nitrile hydratase (NHase) was dissolved in the buffer solution (nitrile hydratase concentration: 2 mg/mL) were prepared.

Next, 1 mL of the buffer solution, 1 mL of the racemase solution, and 10 mg of a support for enzyme immobilization were mixed to prepare a mixed solution. This mixed solution was stored in an incubator at a temperature of 4°C for 24 hours to prepare an immobilized enzyme in which the racemase was adsorbed on the support for enzyme immobilization.

2. Measurement of specific activity of nitrile hydratase in immobilized enzyme

The prepared immobilized enzyme was suspended in 2 mL of a 0.1 M phosphate buffer (pH 7.5). Next, 2 mL of a phosphate buffer containing 20 mM methacrylonitrile (solution for measuring activity) was put in a quartz cuvette, and the cuvette was set in an ultraviolet spectrophotometer.

At the same time when the support suspension containing nitrile hydratase in an amount corresponding to 10 $\mu$g was added to the solution for measuring activity, recording of an increase in the absorbance at a wavelength of 224 nm was started. Specific activity of 1 mg of NHase was calculated on the assumption that the absorption coefficient $\varepsilon$ of methacrylamide to be produced was 2.52 mM$^{-1}$cm$^{-1}$, and activity that catalyzes 1 micromole of the substrate for one minute was 1 unit. FIG. 10 illustrates the specific activity of NHase immobilized on various types of supports.

G-1N exhibited high specific activity corresponding to that of the free enzyme ("Free"), which was not supported on a support.

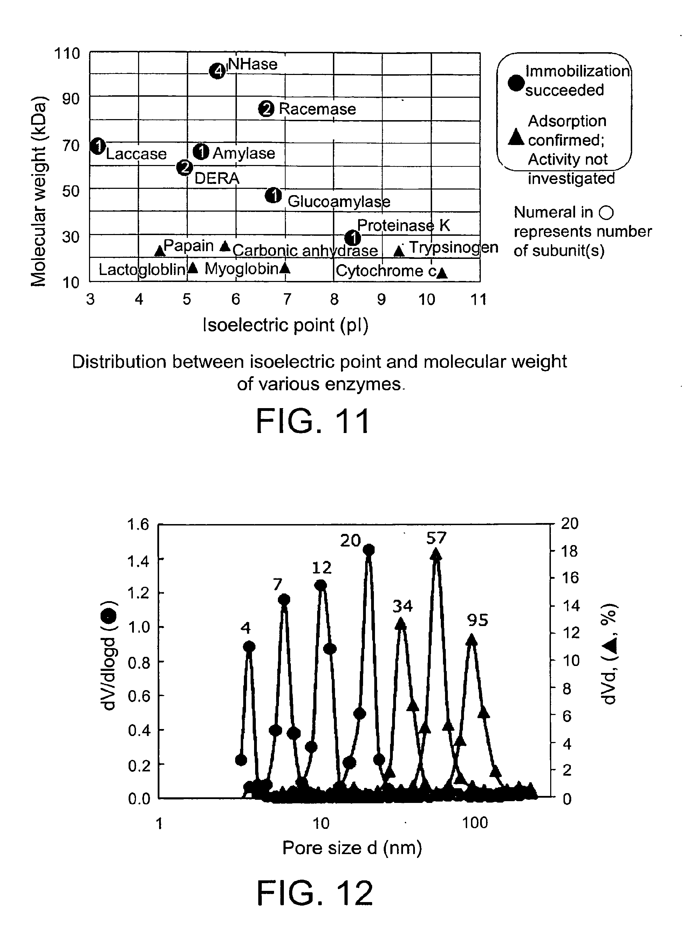

FIG. 11 is a mapping between the isoelectric point and the molecular weight of enzymes, which are indicators representing characteristics of the enzymes, and illustrates data related to the enzymes of Examples 4 to 6, and laccase, glucoamylase, amylase, and protease, all of which were immobilized by the same methods as those described in these Examples, and other enzymes. The number of subunits of each enzyme is also described in FIG. 11. As illustrated in FIG. 11, it was confirmed that all the various types of enzymes were immobilized and high activity could be obtained.

[EXAMPLE 7]

Supports for protein immobilization (porous silica particles) were synthesized using primary particles having various average particle diameters, and the pore size distribution of each of the supports was measured.

1. Supports for protein immobilization (porous silica particles) used in measurement

Methods for producing supports for protein immobilization (porous silica particles) used in the measurement of the pore size distribution in this Example, etc. are described below.

A support for protein immobilization (porous silica particle "X1") having a pore size of 34 nm was prepared as follows. First, 2,000 g of a water-diluted product (silica concentration: 15% by mass) of a silica sol (produced by JGC Catalysts and Chemicals Ltd., "SI-80P", average particle diameter: 80 nm, concentration: 40% by mass) was subjected to a cation exchange, and the pH thereof was adjusted to 2.0. Subsequently, a silicic acid liquid (silica concentration: 4.8% by mass) was added thereto so that a ratio [silica in silica sol]/[silica in silicic acid liquid] = 9/1, and stirring was conducted to prepare a slurry. The solid concentration of the resulting spherical silica fine particle dispersion liquid was adjusted to 15% by mass to prepare a spray liquid. The spray liquid was spray-dried under the conditions of an inlet temperature of 220°C and an outlet temperature of 50°C. Thus, the support for protein immobilization (porous silica particle X1) was obtained.

A support for protein immobilization (porous silica particle "X2") having a pore size of 57 nm was prepared as follows. First, 2,000 g of a water-diluted product (silica concentration: 15% by mass) of a silica sol (produced by JGC Catalysts and Chemicals Ltd., "SS-160", average particle diameter: 160 nm, concentration: 40% by mass) was subjected to a cation exchange, and the pH thereof was adjusted to 2.0. Subsequently, a silicic acid liquid (silica concentration: 4.8% by mass) was added thereto so that a ratio [silica in silica sol]/[silica in silicic acid liquid] = 9/1, and stirring was conducted to prepare a slurry. The solid concentration of the resulting spherical silica fine particle dispersion liquid was adjusted to 15% by mass to prepare a spray liquid. The spray liquid was spray-dried under the conditions of an inlet temperature of 220°C and an outlet temperature of 50°C. Thus, the support for protein immobilization (porous silica particle X2) was obtained.

A support for protein immobilization (porous silica particle "X3") having a pore size of 95 nm was prepared as follows. First, 2,000 g of a water-diluted product (silica concentration: 15% by mass) of a silica sol (produced by JGC Catalysts and Chemicals Ltd., "SS-300", average particle diameter: 300 nm, concentration: 40% by mass) was subjected to a cation exchange, and the pH thereof was adjusted to 2.0. Subsequently, a silicic acid liquid (silica concentration: 4.8% by mass) was added thereto so that a ratio [silica in silica sol]/[silica in silicic acid liquid] = 9/1, and stirring was conducted to prepare a slurry. The solid concentration of the resulting spherical silica fine particle dispersion liquid was adjusted to 15% by mass to prepare a spray liquid. The spray liquid was spray-dried under the conditions of an inlet temperature of 220°C and an outlet temperature of 50°C. Thus, the support for protein immobilization (porous silica particle X3) was obtained.

Table 3 describes characteristics of the porous silica particles X1, X2, and X3.

[Table 3]

[Table 3]

| Porous silica particles X1 to X3 used in Example 7 | | | | | | | | | | | |
|---|---|---|---|---|---|---|---|---|---|---|---|
| Name | Porous silica particles | | | | | | Silica fine particles | | | | |
| | Pore size | Average particle diameter | Specific surface area | Pore volume | Pore size (D1) of peak value in pore size distribution | Ratio of pore volume in (D1)$\times$0.75 to (D1)$\times$1.25 to total maximum volume | Average particle diameter | Sphericity | Coefficient of variation of particle diameter | Whether particle diameter distribution is monodispersion phase or not | Porosity |
| | nm | $\mu$m | m$^2$/g | mL/g | nm | % | nm | - | % | | % |
| X1 | 34.0 | 2.0 | 46 | 0.20 | 34.0 | 78 | 80 | 0.95 | 3 | Monodispersion phase | 33 |
| X2 | 57.0 | 2.0 | 27 | 0.23 | 57.0 | 81 | 160 | 0.94 | 3 | Monodispersion phase | 30 |
| X3 | 95.0 | 2.0 | 20 | 0.21 | 95.0 | 81 | 300 | 0.94 | 3 | Monodispersion phase | 32 |

Furthermore, the porous silica particles P3, P1, P2, and P4 were used as supports for protein immobilization (porous silica particles) having a pore size of 4 nm, 7 nm, 12 nm, and 20 nm, respectively.

Note that the porous silica particles X1 to X3 and the porous silica particles P1 to P4 each have a silanol group on the particle surfaces.

2. Measurement of pore size distributions of porous silica particles

The pore size distribution of each of the supports for protein immobilization (porous silica particles) X1 to X3 and P1 to P4 was measured. In this measurement, for the supports having a pore size of 20 nm or less, the pore volume and the pore size were measured by a nitrogen adsorption method. For the supports having a pore size of more than 20 nm, the pore volume and the pore size were measured by a mercury penetration method.

FIG. 12 illustrates the pore size distributions obtained by the measurement. In FIG. 12, the pore distributions measured by the nitrogen adsorption method and denoted by the curves joining the circular symbols are each represented by a log differential pore volume distribution, i.e., by the relationship of the differential amount of nitrogen gas adsorbed $\Delta V/\Delta(\log(d))$ with respect to the pore size d (nm) (for convenience, represented by "dV/dlogd" in FIG. 12, where V represents the volume of nitrogen gas adsorbed). The pore distributions measured by the mercury penetration method and denoted by the curves joining the triangular symbols are each represented by the relationship of $\Delta Vd$ with respect to the pore size d (for convenience, represented by "dVd" in FIG. 12).

The values written next to the peaks of the graph of FIG. 12 are the averages of the pore sizes of the respective supports.

Most enzyme molecules can be immobilized in pores having a pore size of about 20 nm. However, some enzymes that exhibit activity in the form of a multimer have a size of more than 20 nm. Since the supports for protein immobilization can have a pore size of more than 20 nm as described above, they can immobilize such enzymes that form a multimer having a size exceeding 20 nm. Thus, according to the above results, the present invention can provide a support for protein immobilization (porous silica particle) having a pore with a size suitable for immobilizing such enzymes.

[Industrial Applicability]

The support for protein immobilization according to the present invention is useful as a support of an enzyme or the like.

The immobilized protein according to the present invention realizes, for example, improvement in stability or durability of an enzyme used in various synthesis reactions, improvement in productivity of a target product, continuous production of a target substance, or omission of a step of separating an enzyme from a product in the technical field of a synthesis reaction using an enzyme, e.g., chemical synthesis, synthesis of fine chemicals, synthesis of medicine, or production of food. Furthermore, the immobilized protein according to the present invention can be applied to various types of enzymes.

**Claims**

1. A support for protein immobilization, comprising:

   porous silica particles each having an interparticle gap structure inside,
   wherein the porous silica particles satisfy (1) to (6) below and have a silanol group, an anion-exchange group, or a cation-exchange group on the surfaces thereof:

   (1) an average particle diameter (Da) is in the range of 0.5 to 100 $\mu$m;
   (2) a specific surface area is in the range of 10 to 250 $m^2$/g;
   (3) a pore volume (Pv) is in the range of 0.10 to 0.32 mL/g;
   (4) a pore size (Pms) of a peak value in a pore size distribution (X axis: pore size [Ps], Y axis: value obtained by differentiating pore volume with respect to pore size) is in the range of 2 to 200 nm;
   (5) a total volume of pores having a pore size in the range of (Pms) $\times$ 0.75 nm to (Pms) $\times$ 1.25 nm is 70% or more of a total volume of all pores; and
   (6) a porosity is in the range of 5% to 50%.

2. The support for protein immobilization according to claim 1,
   wherein the porous silica particles are composed of spherical aggregates each obtained by aggregating spherical silica fine particles which have an average particle diameter (Db) of 10 to 500 nm, a sphericity in the range of 0.9 to 1, and a coefficient of variation (CV value) of the particle diameter in the range of 2% to 10%, and whose particle diameter distribution exhibits a monodispersion phase.

3. A support for protein immobilization, comprising:

porous silica particles each having an interparticle gap structure inside,
wherein the porous silica particles satisfy (1) to (6) below and have a silanol group, an anion-exchange group, or a cation-exchange group on the surfaces thereof:

(1) an average particle diameter (Da) is in the range of 0.5 to 50 $\mu$m;
(2) a specific surface area is in the range of 10 to 250 $m^2/g$;
(3) a pore volume (Pv) is in the range of 0.10 to 0.32 mL/g;
(4) a pore size (Pms) of a peak value in a pore size distribution (X axis: pore size [Ps], Y axis: value obtained by differentiating pore volume with respect to pore size) is in the range of 2 to 50 nm;
(5) a total volume of pores having a pore size in the range of (Pms) $\times$ 0.75 nm to (Pms) $\times$ 1.25 nm is 80% or more of a total volume of all pores; and
(6) a porosity is in the range of 5% to 50%.

4. The support for protein immobilization according to claim 3,
wherein the porous silica particles are composed of spherical aggregates each obtained by aggregating spherical silica fine particles which have an average particle diameter (Db) of 10 to 50 nm, a sphericity in the range of 0.9 to 1, and a coefficient of variation (CV value) of the particle diameter in the range of 2% to 10% and whose particle diameter distribution exhibits a monodispersion phase.

5. The support for protein immobilization according to any one of claims 1 to 4, wherein the anion-exchange group is a substituent having a structure containing an amino group or a quaternary ammonium group.

6. The support for protein immobilization according to any one of claims 1 to 4, wherein the cation-exchange group is a substituent having a structure containing a group selected from a carboxyl group, a phosphate group, and a sulfoxyl group.

7. The support for protein immobilization according to any one of claims 1 to 6, wherein the porous silica particles are obtained by conducting a surface treatment with a silane coupling agent or an organic acid.

8. The support for protein immobilization according to any one of claims 1 to 7, wherein the porous silica particles are obtained by treating porous silica particles with an amino group-containing silane coupling agent, and further subjecting the porous silica particles to a surface treatment with an organic acid.

9. The support for protein immobilization according to any one of claims 1 to 8, wherein the support is used for immobilizing an enzyme.

10. The support for protein immobilization according to any one of claims 1 to 8, wherein the support is used for immobilizing a multienzyme.

11. An immobilized protein obtained by immobilizing a protein on the support for protein immobilization according to any one of claims 1 to 8.

12. The immobilized protein according to claim 11, wherein the protein is an enzyme.

13. The immobilized protein according to claim 12, wherein the enzyme is a racemase.

14. The immobilized protein according to claim 12, wherein the enzyme is deoxyriboaldolase.

15. A method for producing an immobilized protein, comprising a step of allowing a protein to adsorb onto the support for protein immobilization according to any one of claims 1 to 8.

16. The method according to claim 15, wherein the protein is an enzyme.

17. The method for producing an immobilized protein according to claim 15 or 16, wherein the protein is allowed to adsorb onto the support for protein immobilization in a buffer solution in the range of 4°C to 25°C.

FIG. 1

Change in specific activity of racemase in repeated uses

FIG. 2

Change in specific activity of racemase in repeated uses

# FIG. 3

Reaction with mediation of deoxyriboaldolase

# FIG. 4

Fig. 1. DERA-catalyzed tandem aldol reaction
Lactone synthesis reaction by a tandem aldol reaction
catalyzed by DERA

## FIG. 5

Specific activity of immobilized DERA in forward
reaction

## FIG. 6

Change in specific activity of DERA in forward reaction in
acetaldehyde (300 mM)

FIG. 7

Amount of product of reverse reaction by DERA in acetaldehyde (300 mM)

FIG. 8

Comparison of amount of produced lactone

FIG. 9

Specific activities of NHase immobilized on various types of supports

FIG. 10

Distribution between isoelectric point and molecular weight
of various enzymes.

FIG. 11

FIG. 12

**INTERNATIONAL SEARCH REPORT**

| International application No. |
| --- |
| PCT/JP2011/063160 |

A.   CLASSIFICATION OF SUBJECT MATTER
*C12N11/14*(2006.01)i, *C01B37/00*(2006.01)i, *C07K17/14*(2006.01)i

According to International Patent Classification (IPC) or to both national classification and IPC

B.   FIELDS SEARCHED

Minimum documentation searched (classification system followed by classification symbols)
C12N11/14, C01B37/00, C07K17/14

Documentation searched other than minimum documentation to the extent that such documents are included in the fields searched
Jitsuyo Shinan Koho          1922-1996     Jitsuyo Shinan Toroku Koho     1996-2011
Kokai Jitsuyo Shinan Koho   1971-2011     Toroku Jitsuyo Shinan Koho     1994-2011

Electronic data base consulted during the international search (name of data base and, where practicable, search terms used)
JSTPlus/JMEDPlus/JST7580(JDreamII), WPI, CAplus(STN)

C.   DOCUMENTS CONSIDERED TO BE RELEVANT

| Category* | Citation of document, with indication, where appropriate, of the relevant passages | Relevant to claim No. |
| --- | --- | --- |
| A | JP 61-174103 A  (Catalysts & Chemicals Industries Co., Ltd.), 05 August 1986 (05.08.1986), entire text (Family: none) | 1-17 |
| A | JP 61-168503 A  (Catalysts & Chemicals Industries Co., Ltd.), 30 July 1986 (30.07.1986), entire text (Family: none) | 1-17 |
| P,X | JP 2010-138021 A  (JGC Catalysts and Chemicals Ltd.), 24 June 2010 (24.06.2010), examples 1 to 4; paragraph [0065] (Family: none) | 1-17 |

☐  Further documents are listed in the continuation of Box C.          ☐  See patent family annex.

| | |
| --- | --- |
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |
| Date of the actual completion of the international search<br>    21 June, 2011 (21.06.11) | Date of mailing of the international search report<br>    05 July, 2011 (05.07.11) |
| Name and mailing address of the ISA/<br>    Japanese Patent Office | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10328558 A
- JP 2006232594 A
- JP 2009073681 A
- JP 2001170500 A
- JP 2007051076 A
- JP 2009125006 A
- JP 2008024567 A
- JP 2007076941 A
- JP 2009153448 A
- JP 2001178457 A
- JP 2002262863 A
- JP 5132309 A
- JP 10454043 A
- JP 7133105 A
- JP 3043201 A
- JP 2061406 A
- JP 2004083501 A